# EUROPEAN PATENT APPLICATION

(11) **EP 2 327 713 A1**
(43) Date of publication of application: **01.06.2011**
(21) Application number: 10183238.4
(22) Date of filing: 29.09.2000
(51) Int. Cl.: C07K 5/10, C07K 14/76, C07K 14/795

(54) **Metal-binding compounds and uses therefor**

(30) Priority: 01.10.1999 US 157404 P; 13.06.2000 US 211078 P
(62) Divisional of application: 00967163.7
(71) Applicant: DMI Biosciences, Inc., Englewood, CO 80110 (US)
(72) Inventor: Bar-Or, David, Englewood, CO 80110 (US); Curtis, Gerald, C, Cardiff, CF2 5HA (GB); Lau, Edward, Boulder, CO 80301 (US); Rao, Nagaraja K.R., Cardiff, CF4 4QN (GB); Winkler, James, V, Denver, CO 80206 (US); Crook, Wannell, M, Castle Rock, CO 80104 (US)
(74) Representative: Neilson, Martin Mark

(57) **Abstract**

The invention provides a method of reducing the damage done by reactive oxygen species (ROS) in an animal. The invention also provides a method of reducing the concentration of a metal in an animal. These methods comprise administering to the animal an effective amount of a metal-binding compound as further described in the application. The invention further provides a method of reducing the damage done by ROS in a tissue or an organ that has been removed from an animal. This method comprising contacting the tissue or organ with a solution containing an effective amount of a metal-binding compound of the invention. The invention further provides novel metal-binding compounds, pharmaceutical compositions comprising the metal-binding compounds, and kits comprising a container holding a metal-binding compound of the invention.

## Description

### FIELD OF THE INVENTION

The invention relates to a method of reducing the molecular, cellular and tissue damage done by reactive oxygen species (ROS). The invention also relates to certain compounds, especially certain peptides and peptide derivatives, that bind metal ions, particularly Cu(II). The binding of metal ions by the compounds of the invention inhibits the formation and/or accumulation of ROS and/or targets the damage done by ROS to the compounds themselves (i. e., the compounds of the invention may act as sacrificial antioxidants).

### BACKGROUND

Reactive oxygen species (ROS) include free radicals (e.g., superoxide anion and hydroxyl, peroxyl, and alkoxyl radicals) and non-radical species (e.g., singlet oxygen and hydrogen peroxide). ROS are capable of causing extensive cellular and tissue damage, and they have been reported to play a maj or role in a variety of diseases and conditions. Indeed, ROS have been implicated in over 100 diseases and pathogenic conditions, and it has been speculated that ROS may constitute a common pathogenic mechanism involved in all human diseases. Stohs, J. Basic Clin. Physiol. Pharmacol., 6, 205-228 (1995). For reviews describing ROS, their formation, the mechanisms by which they cause cellular and tissue damage, and their involvement in numerous diseases and disorders, see, e.g., Manso, Rev. Port. Cardiol., 11, 997-999 (1992); Florence, Aust. N Z J. Opthalmol., 23, 3-7 (1992); Stohs, J. Basic Clin. Physiol. Pharmacol., 6, 205-228 (1995); Knight, Ann. Clin. Lab. Sci., 25, 111-121 (1995); Kerr et al., Heart & Lung, 25, 200-209 (1996); Roth, Acta Chir. Hung., 36, 302-305 (1997).

Ischemia/reperfusion is the leading cause of illness and disability in the world. Cardiovascular ischemia, in which the body's capacity to provide oxygen to the heart is diminished, is the leading cause of illness and death in the United States. Cerebral ischemia is a precursor to cerebrovascular accident (stroke), which is the third leading cause of death in the United States. Ischemia also occurs in other organs (e.g., kidney, liver, lung, and the intestinal tract), in harvested organs ( e.g., organs harvested for transplantation or for research (e.g., perfused organ models)), and as a result of surgery where blood flow is interrupted (e.g., open heart surgery and coronary bypass surgery). Ischemia need not be limited to one organ; it can also be more generalized (e.g., in hemorrhagic shock).

Cellular and tissue damage occur during ischemia as result of oxygen deficiency. However, the damage that occurs during ischemia is generally light compared to the severe damage that occurs upon reperfusion of ischemic tissues and organs. See, *e.g.,* Manso, Rev. Port. Cardiol., 11, 997-999 (1992); Stohs, J. Basic Clin. Physiol. Pharmacol., 6, 205-228 (1995); Knight, Ann. Clin. Lab. Sci., 25, 111-121 (1995); Kerr et al., Heart & Lung, 25, 200-209 (1996); Roth, Acta Chir. Hung., 36, 302-305 (1997). ROS have been reported to be responsible for the severe damage caused by reperfusion of ischemic tissues and organs. See, e.g., Manso, Rev. Port. Cardiol., 11, 997-999 (1992); Stohs, J. Basic Clin. Physiol. Pharmacol., 6, 205-228 (1995); Knight, Ann. Clin. Lab. Sci., 25, 111-121 (1995); Kerr et al., Heart & Lung, 25, 200-209 (1996); Roth, Acta Chir. Hung., 36, 302-305 (1997).

Metal ions, primarily transition metal ions, can cause the production and accumulation of ROS. In particular, copper and iron ions released from storage sites are one of the main causes ofthe production of ROS following injury, including ischemia/reperfusion injury and injury due to heat, cold, trauma, excess exercise, toxins, radiation, and infection. Roth, Acta Chir. Hung., 36, 302-305 (1997). Copper and iron ions, as well as other transition metal ions (e.g., vanadium, and chromium ions), have been reported to catalyze the production of ROS. *See, e.g.,* Stohs, J. Basic Clin. Physiol. Pharmacol., 6, 205-228 (1995); Halliwell et al., Free Radicals In Biology And Medicine, pages 1-19 (Oxford University 1989); Marx et al., Biochem. J., 236, 397-400 (1985); Quinlan et al., J. Pharmaceutical Sci., 81, 611-614 (1992). Other transition metal ions *(e.g.,* cadmium, mercury, and nickel ions) and other metal ions (e.g., arsenic and lead ions) have been reported to deplete some of the molecules of the natural antioxidant defense system, thereby causing an increased accumulation of ROS. *See, e.g.,* Stohs, J. Basic Clin. Physiol. Pharmacol., 6, 205-228 (1995). Although it has been reported that free copper ions bind nonspecifically to the amino groups of essentially any protein (Gutteridge et al., Biochim. Biophys. Acta, 759, 38-41 (1983)), copper ions bound to proteins can still cause the production of ROS which damage at least the protein to which the copper ions are bound. *See, e.g.,* Gutteridge et al., Biochim. Biophys. Acta, 759, 38-41 (1983); Marx et al., Biochem. J., 236, 397-400 (1985); Quinlan et al., J. Pharmaceutical Sci., 81, 611-614 (1992).

Albumin has been characterized as an extracellular antioxidant. See, e.g., Halliwell and Gutteridge, Arch. Biochem. Biophys., 280,1-8 (1990); Das et al., Methods Enzymol., 233, 601-610 (1994); Stohs, J. Basic Clin. Physiol. Pharmacol., 6, 205-228 (1995); Dunphy et al., Am. J. Physiol., 276, H1591-H1598 (1999)). The antioxidant character of albumin has been attributed to several of albumin's many physiological functions, including albumin's ability to bind metals (particularly copper ions), to bind fatty acids, to bind and transport steroids, to bind and transport bilirubin, to scavenge HOCl, and others. See, e.g., Halliwell and Gutteridge, Arch. Biochem. Biophys., 280, 1-8 (1990); Halliwell and Gutteridge, Arch. Biochem. Biophys., 246, 501-514 (1986); Stohs, J. Basic Clin. Physiol. Pharmacol., 6, 205-228 (1995); Dunphy et al., Am. J. Physiol., 276, H1591-H 1598(1999)). Albumin contains several metal binding sites, including one at the N-terminus. The N-terminal metal-binding sites of several albumins, including human, rat and bovine serum albumins, exhibit high-affinity for Cu(II) and Ni(II), and the amino acids involved in the high-affinity binding of these metal ions have been identified. *See* Laussac et al., Biochem., 23, 2832-2838 (1984); Predki et al., Biochem. J., 287, 211-215 (1992); Masuoka et al., J. Biol. Chem., 268, 21533-21537 (1993). It has been reported that copper bound to albumin at metal binding sites other than the high-affinity N-terminal site produce free radicals which causes extensive damage to albumin at sites dictated by the location of the "loose" metal binding sites, resulting in the characterization of albumin as a "sacrificial antioxidant." See Marx et al., Biochem. J., 236, 397-400 (1985); Halliwell et al., Free Radicals In Biology And Medicine, pages 1-19 (Oxford University 1989); Halliwell and Gutteridge, Arch. Biochem. Biophys., 280, 1-8 (1990); Quinlan et al., J. Pharmaceutical Sci., 81, 611-614 (1992).

Despite the foregoing, attempts to use albumin as a treatment for cerebral ischemia have shown mixed results. It has been reported that albumin is, and is not, neuroprotective in animal models of cerebral ischemia. Compare Huh et al., Brain Res., 804,105-113 (1998) and Remmers et al., Brain Res., 827, 237-242 (1999), with Little et al., Neurosurgery, 9, 552-558 (1981) and Beaulieu et al., J. Cereb. Blood Flow. Metab., 18, 1022-1031 (1998).

Mixed results have also been obtained using albumin in cardioplegia solutions for the preservation of excised hearts. As reported in Dunphy et al., Am. J. Physiol., 276, H1591-H1598 (1999), the addition of albumin to a standard cardioplegia solution for the preservation of excised hearts did not improve the functioning of hearts perfused with the solution for twenty-four hours. Hearts did demonstrate improved functioning when perfused with a cardioplegia solution containing albumin and several enhancers (insulin, ATP, corticosterone, and pyruvic acid). This was a synergistic effect, since the enhancers alone, as well as the albumin alone, did not significantly improve heart function. An earlier report of improved heart function using cardioplegia solutions containing albumin was also attributed to synergism between enhancers and albumin. See the final paragraph of Dunphy et al., Am. J. Physiol., 276, H1591-H1598 (1999) and Hisatomi et al., Transplantation, 52, 754-755 (1991), cited therein. In another study, hearts perfused with a cardioplegia solution containing albumin increased reperfusion injury in a dose-related manner, as compared to a solution not containing albumin. Suzer et al., Pharmacol. Res., 37, 97-101 (1998). Based on their study and the studies of others, Suzer et al. concluded that albumin had not been shown to be effective for cardioprotection. They further noted that the use of albumin in cardioplegia solutions could be unsafe due to possible allergic reactions and the risks associated with the use of blood products.

Finally, although albumin has been characterized as an antioxidant, it has also been reported to enhance superoxide anion production by microglia (Si et al., *GLIA,* **21**, 413-418 (1997)). This result led the authors to speculate that albumin leaking through the disrupted blood brain barrier in certain disorders potentiates the production of superoxide anion by microglia, and that this increased production of superoxide anion is responsible for the pathogenesis of neuronal damage in cerebral ischemia/reperfusion and some neurodegenerative diseases.

As noted above, the N-terminal metal-binding sites of several albumins exhibit high-affinity for Cu(II) and Ni(II). These sites have been studied extensively, and a general amino terminal Cu(II)- and Ni(II)-binding (ATCUN) motif has been identified. See, e.g., Harford and Sarkar, Acc. Chem. Res., 30, 123-130 (1997). The ATCUN motif can be defined as being present in a protein or peptide which has a free -NH₂ at the N-terminus, a histidine residue in the third position, and two intervening peptide nitrogens. See, e.g., Harford and Sarkar,Acc. Chem. Res., 30,123-130 (1997). Thus, the ATCUN motif is provided by the peptide sequence Xaa Xaa His, where Xaa is any amino acid except proline. See, e.g., Harford and Sarkar, Acc. Chem. Res., 30, 123-130 (1997). The Cu(II) and Ni(II) are bound by four nitrogens provided by the three amino acids of the ATCUN motif (the nitrogen of the free -NH₂, the two peptide nitrogens, and an imidazole nitrogen of histidine) in a slightly distorted square planar configuration. See, e.g., Harford and Sarkar, Acc. Chem. Res., 30,123-130 (1997). Side-chain groups ofthe three amino acids ofwhich the ATCUN motif consists can be involved in the binding of the Cu(II) and Ni(II), and amino acids near these three N-terminal amino acids may also have an influence on the binding of these metal ions. See, e.g., Harford and Sarkar, Acc. Chem. Res., 30, 123-130 (1997); Bal et al., Chem. Res. Toxicol., 10, 906-914 (1997). For instance, the sequence of the N-terminal metal-binding site of human serum albumin is Asp Ala His Lys [SEQ ID NO:1], and the free side-chain carboxyl of the N-terminal Asp and the Lys residue have been reported to be involved in the binding of Cu(II) and Ni(II), in addition to the four nitrogens provided by Asp Ala His. See Harford and Sarkar, Acc. Chem. Res., 30, 123-130 (1997); Laussac et al., Biochem., 23, 2832-2838 (1984); and Sadler et al., Eur. J. Biochem., 220, 193-200 (1994).

The ATCUN motif has been found in other naturally-occurring proteins besides albumins, and non-naturally-occurnng peptides and proteins comprising the ATCUN motif have been synthesized. See, *e.g.,* Harford and Sarkar,Acc. Chem. Res., 30,123-130 (1997); Bal et al., Chem. Res. Toxicol., 10, 906-914 (1997); Mlynarz, et al., Speciation 98: Abstracts*,* http://www.jate.u-szeged.hu/∼spec98/abstr/mlynar.html. Cu(II) andNi(II) complexes of ATCUN-containing peptides and proteins have been reported to exhibit superoxide dismutase (SOD) activity. See Cotelle et al., J. Inorg. Biochem., 46, 7-15 (1992); Ueda et al., J. Inorg. Biochem., 55, 123-130 (1994). Despite their reported SOD activity, these complexes still produce free radicals which damage DNA, proteins and other biomolecules. See Harford and Sarkar, Acc. Chem. Res., 30, 123-130 (1997); Bal et al., Chem. Res. Toxicol., 10, 915-21 (1997); Ueda et al., Free Radical Biol. Med., 18, 929-933 (1995); Ueda et al., J. Inorg. Biochem., 55, 123-130 (1994); Cotelle et al., J. Inorg. Biochem., 46, 7-15 (1992). As a consequence, it has been hypothesized that at least some of the adverse effects of copper and nickel *in vivo* are attributable to the binding of Cu(II) and Ni(II) to ATCUN-containing proteins which causes the production of damaging free radicals. See Harford and Sarkar, Acc. Chem. Res., 30, 123-130 (1997); Bal et al., Chem. Res. Toxicol., 10, 915-921 (1997); Cotelle et al., J. Inorg. Biochem., 46, 7-15 (1992). *Cf.* Koch et al., Chem. & Biol., 4, 549-60 (1997). The damaging effects produced by a Cu(II) complex of an ATCUN-containing peptide have been exploited to kill cancer cells *in vitro* and to produce anti-tumor *effects in vivo.* See Harford and Sarkar, Acc. Chem. Res., 30, 123-130 (1997).

### SUMMARY OF THE INVENTION

The invention provides a method of reducing the damage done by reactive oxygen species (ROS) in an animal. The method comprises administering to the animal an effective amount of a metal-binding peptide having the formula P₁ - P₂ or a physiologically-acceptable salt thereof.

The invention further provides a method of reducing the damage done by ROS in a tissue or an organ that has been removed from an animal. This method comprises contacting the tissue or organ with a solution containing an effective amount of the peptide P₁ - P₂ or a physiologically-acceptable salt thereof.

The invention also provides a method of reducing the concentration of a metal in an animal in need thereof. The method comprises administering to the animal an effective amount of a metal-binding peptide having the formula P₁ - P₂ or a physiologically-acceptable salt thereof.

The invention also provides a pharmaceutical composition comprising a pharmaceutically-acceptable carrier and the peptide P₁ - P₂ or a physiologically-acceptable salt thereof.

In addition, the invention provides a kit for reducing the damage done by ROS in a tissue or organ that has been removed from an animal. The kit comprises a container holding the peptide P₁ - P₂.

In the formula P₁ - P₂:
P₁ is Xaa₁ Xaa₂ His or Xaa₁ Xaa₂ His Xaa₃; and
P₂ is (Xaa₄)n.

Xaa₁ is glycine (Gly), alanine (Ala), valine (Val), leucine (Leu), isoleucine (Ile), serine (Ser), threonine (Thr), aspartic acid (Asp), asparagine (Asn), glutamic acid (Glu), glutamine (Gln), lysine (Lys), hydroxylysine (Hylys), histidine (His), arginine (Arg), ornithine (Orn) phenylalanine (Phe), tyrosine (Tyr), tryptophan (Trp), cysteine (Cys), methionine (Met) or α-hydroxymethylserine (HMS). Xaa₁ is preferably Asp, Glu, Arg, or HMS. More preferably, Xaa₁ is Asp or Glu. Most preferably Xaa₁ is Asp.

Xaa₂ is Gly, Ala, β-Ala, Val, Leu, Ile, Ser, Thr, Asp, Asn, Glu, Gln, Lys, Hylys, His, Arg, Om, Phe, Tyr, Trp, Cys, Met or HMS. Xaa₂ is preferably Gly, Ala, Val, Leu, Ile, Thr, Ser, Asn, Met, His or HMS. More preferably Xaa₂ is Ala, Val, Thr, Ser, or HMS. Even more preferably Xaa₂ is Ala, Thr, or HMS. Most preferably Xaa₂ is Ala.

Xaa₃ is Gly, Ala, Val, Lys, Arg, Om, Asp, Glu, Asn, Gln, or Trp, preferably Lys. Xaa₄ is any amino acid.

Finally, n is 0-100, preferably 0-10, more preferably 0-5, and most preferably 0.

In a preferred embodiment, at least one of the amino acids of P₁, other than β-Ala when it is present, is a D-amino acid. Preferably, the D-amino acid is Xaa₁, His, or both. Most preferably all of the amino acids of P₁, other than β-Ala, are D-amino acids. In another preferred embodiment, at least one of the amino acids of P₁ other than β-Ala is a D-amino acid, and at least 50% of the amino acids of P₂ are also D-amino acids. Most preferably all of the amino acids of P₂ are D-amino acids.

In another preferred embodiment, at least one amino acid of P₁ and/or P₂ is substituted with (a) a substituent that increases the lipophilicity of the peptide without altering the ability of P₁ to bind metal ions, (b) a substituent that protects the peptide from proteolytic enzymes without altering the ability of P₁ to bind metal ions, or (c) a substituent which is a non-peptide, metal-binding functional group that improves the ability of the peptide to bind metal ions.

The invention provides another method of reducing the damage done by ROS in an animal. The method comprises administering to the animal an effective amount of a metal-binding peptide (MBP) having attached thereto a non-peptide, metal-binding functional group. The metal-binding peptide MBP may be any metal-binding peptide, not just P₁ - P₂.

The invention further provides another method of reducing the damage done by ROS in a tissue or an organ that has been removed from an animal. This method comprises contacting the tissue or organ with a solution containing an effective amount of a metal-binding peptide MBP having attached thereto a non-peptide, metal-binding functional group.

The invention provides another method of reducing the concentration of a metal in an animal in need thereof. The method comprises administering to the animal an effective amount of a metal-binding peptide MBP having attached thereto a non-peptide, metal-binding functional group.

The invention also provides a pharmaceutical composition comprising a pharmaceutically-acceptable carrier and a metal-binding peptide MBP having attached thereto a non-peptide, metal-binding functional group.

The invention also provides a kit for reducing the damage done by ROS in a tissue or organ that has been removed from an animal. The kit comprises a container holding a metal-binding peptide MBP having attached thereto a non-peptide, metal-binding functional group.

The invention provides yet another method of reducing the damage done by reactive oxygen species (ROS) in an animal. The method comprises administering to the animal an effective amount of a metal-binding peptide dimer of the formula P₃ - L - P₃, wherein each P₃ may be the same or different and is a peptide which is capable of binding a metal ion, and L is a chemical group which connects the two P₃ peptides through their C-terminal amino acids. In a preferred embodiment, one or both of the two P₃ peptides is P₁.

The invention further provides a method of reducing the damage done by ROS in a tissue or an organ that has been removed from an animal. This method comprises contacting the tissue or organ with a solution containing an effective amount of the metal-binding peptide dimer of the formula P₃ - L - P₃.

The invention also provides a method of reducing the concentration of a metal in an animal in need thereof. The method comprises administering to the animal an effective amount of the metal-binding peptide dimer of the formula P₃ - L - P₃.

The invention also provides a pharmaceutical composition comprising a pharmaceutically-acceptable carrier and the metal-binding peptide dimer of the formula P₃ - L - P₃.

In addition, the invention provides a kit for reducing the damage done by ROS in a tissue or organ that has been removed from an animal. The kit comprises a container holding the metal-binding peptide dimer of the formula P₃ - L - P₃.

In addition, the invention provides a peptide having the formula P₁ - P₂, or a physiologically-acceptable salt thereof, wherein at least one amino acid of P₁, other than β-Ala, is a D-amino acid.

Further provided by the invention is a peptide having the formula P₁ - P₂, or a physiologically-acceptable salt thereof, wherein at least one amino acid of P₁ and/or P₂ is substituted with (a) a substituent that increases the lipophilicity of the peptide without altering the ability of P₁ to bind metal ions, (b) a substituent that protects the peptide from proteolytic enzymes without altering the ability of P₁ to bind metal ions, or (c) a substituent which is a non-peptide, metal-binding functional group that improves the ability of the peptide to bind metal ions.

In addition, the invention provides a peptide having the formula P₁ - P₂, wherein P₁ is defined above, and P₂ is a peptide sequence which comprises the sequence of a metal-binding site. In particular, P₂ may have one of the following sequences: (Xaa₄)ₘ Xaa₃ His Xaa₂ Xaa₅, (Xaa₄)ₘ His Xaa₂ Xaa₅, (Xaa₄)ₘ Xaa₅ Xaa₂ His Xaa₃, or (Xaa₄)ₘ Xaa₅ Xaa₂ His, where Xaa₅ is an amino acid having a free side-chain -NH₂ and m is 0-5.

The invention also provides a metal-binding peptide MBP having attached thereto a non-peptide, metal-binding functional group.

Finally, the invention provides the metal-binding peptide dimer of the formula P₃ - L - P₃.

### BRIEF DESCRIPTION OF THE DRAWINGS

Figures 1A-D: Formulas of tetrapeptide Asp Ala His Lys [SEQ ID NO: 1] showing points of possible substitution.
Figures 2A-B: Schematic diagrams of the synthesis of derivatives of the tetrapeptide Asp Ala His Lys [SEQ ID NO:1] coming within the formula of Figure 1C (Figure 2A) and Figure 1B (Figure 2B).
Figure 3A-B: Formulas of cyclohexane diamine derivatives.
Figures 3C-D: Schematic diagrams of syntheses of cyclohexane diamine derivatives of the tetrapeptide Asp Ala His Lys [SEQ ID NO:1].
Figure 4: Formula of a tetraacetic acid derivative of the tetrapeptide Asp Ala His Lys [SEQ ID NO:1].
Figure 5 : Formula of a bispyridylethylamine derivative ofthe tetrapeptide Asp Ala His Lys [SEQ ID NO:1].
Figures 6A-B: Formulas of mesoporphyrin IX with (Figure 6B) and without (Figure 6A) a bound metal ion M.
Figure 6C: Formula of mesoporphyrin IX derivative of the tetrapeptide Asp Ala His Lys [SEQ ID NO:1].
Figure 7: Formulas of monosaccharides.
Figure 8: Diagram of a parabiotic blood perfusion system in which an isolated heart is perfused in the Langendorff mode with blood at 37°C from a support animal of the same species.
Figure 9: Diagram of the treatments of isolated perfused hearts with drug and saline in the parabiotic blood perfusion system illustrated in Figure 8.
Figure 10: Graph of contracture versus duration of ischemia showing the effect of a drug (D-Asp D-Ala D-His D-Lys) on contracture during ischemia in the blood-perfused rat heart model illustrated in Figures 8 and 9. In Figure 10, -□- is saline control, and -O- is drug.
Figure 11: Graph of left ventricle diastolic pressure (LVDP; expressed as a percentage of the 20-minute pre-intervention baseline value) versus duration of reperfusion showing the effect of the drug D-Asp D-Ala D-His D-Lys on post-ischemic recovery of LVDP in the blood-perfused rat heart model illustrated in Figures 8 and 9. * indicates
   p ≤ 0.05. In Figure 11, -□- is saline control, and -O- is drug.
Figure 12: Graph of left ventricle end diastolic pressure (LVEDP) versus duration of reperfusion showing the effect of the drug D-Asp D-Ala D-His D-Lys on post-ischemic recovery of LVEDP in the blood-perfused rat heart model illustrated in Figures 8 and 9. * indicates p ≤ 0.05. In Figure 12, -□- is saline control, and -O- is drug.
Figure 13 : Graph of heart rate (expressed as a percentage of the 20-minute pre-intervention baseline value) versus duration of reperfusion showing the effect of the drug D-Asp D-Ala D-His D-Lys on post-ischemic recovery of heart rate in the blood-perfused rat heart model illustrated in Figures 8 and 9. * indicates p ≤ 0.05. In Figure 13, -□- is saline control, and -O- is drug.
Figure 14: Graph of perfusion pressure (expressed as a percentage of the 20-minute pre-intervention baseline value) versus duration of reperfusion showing the effect of the drug D-Asp D-Ala D-His D-Lys on post-ischemic recovery of perfusion pressure in the blood-perfused rat heart model illustrated in Figures 8 and 9. In Figure 14, -□- is saline control, and -O- is drug.
Figure 15A-B: Graphs of absorbance at 532 nm (A532) versus incubation time in an assay for the production of hydroxyl radicals. In Figure 15A, ■ = ascorbate only, ◆ = copper and ascorbate, A = tetrapeptide (L-Asp L-Ala L-His L-Lys [SEQ ID NO:1]), copper and ascorbate (tetrapeptide/copper ratio of 1:1), X = tetrapeptide, copper and ascorbate (tetrapeptide/copper ratio of 2:1). In Figure 15B, ◆ = copper and ascorbate and ■ = tetrapeptide, copper and ascorbate (tetrapeptide/copper ratio of 2:1).
Figure 16: Graph of % inhibition versus concentration tetrapeptide (L-Asp L-Ala L-His L-Lys [SEQ ID NO:1])-copper complex at a tetrapeptide/copper ratio of 1:1 in the xanthine oxidase assay for superoxide dismutase activity.
Figure 17: Graph of absorbance at 560 nm (A560) versus time in an assay for superoxide radical production. In Figure 17, ■ = ascorbate only, ◆ = copper and ascorbate, Δ = tetrapeptide (L-Asp L-Ala L-His L-Lys [SEQ ID NO:1]), copper and ascorbate (tetrapeptide/copper ratio of 1:1), X = tetrapeptide, copper and ascorbate (tetrapeptide/copper ratio of 2:1).

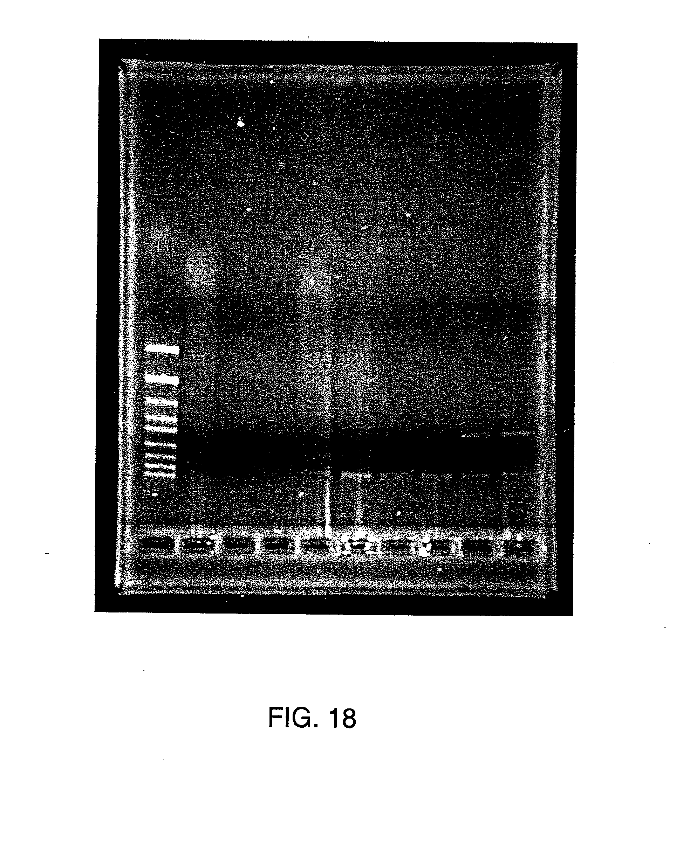

Figure 18: Gel after electrophoresis of DNA treated in various ways. Lane 1 - 17 µg/ml plasmid DNA (untreated control); Lane 2 -17 µg/ml plasmid DNA and 50 µM CuCl₂; Lane 3 -17 µg/ml plasmid DNA and 2.5 mM ascorbate; Lane 4 -17 µg/ml plasmid DNA, 2.5 mM ascorbate, 50 µM CuCl₂, and 200 µM tetrapeptide (L-Asp L-Ala L-His L-Lys [SEQ ID NO:1]) (4:1 ratio tetrapeptide/copper); Lane 5 - 17 µg/ml plasmid DNA, 2.5 mM ascorbate, 50 µM CuCl₂, and 100 µM tetrapeptide (2:1 ratio tetrapeptide/copper); Lane 6 -17 µg/ml plasmid DNA, 2.5 mM ascorbate, 50 µM CuCl₂, and 50 µM tetrapeptide (1:1 ratio tetrapeptide/copper); Lane 7 - 17 µg/ml plasmid DNA, 2.5 mM ascorbate, 50 µM CuCl₂, and 25 µM tetrapeptide (1:2 ratio tetrapeptide/copper); Lane 8 - 17 µg/ml plasmid DNA, 2.5 mM ascorbate, 50 µM CuCl₂, and 12.5 µM tetrapeptide (1:4 ratio tetrapeptide/copper); Lane 9 - 17 µg/ml plasmid DNA, 2.5 mM ascorbate, and 50 µM CuCl₂ (positive control); and Lane 10 - DNA ladder.
Figure 19A: Formulas of peptide dimers according to the invention.
Figures 19B-C: Diagrams illustrating the synthesis of peptide dimers according to the invention.

### DETAILED DESCRIPTION OF THE

### PRESENTLY-PREFERRED EMBODIMENTS

The invention provides a peptide of the formula P₁ - P₂. P₁ is Xaa₁ Xaa₂ His or is Xaa₁ Xaa₂ His Xaa₃, wherein Xaa₁, Xaa₂, and Xaa₃ are defined above. P₁ is a metal-binding peptide sequence that binds transition metal ions of Groups 1 *b-7b* or 8 of the Periodic Table of elements (including V, Co, Cr, Mo, Mn, Ba, Zn, Hg, Cd, Au, Ag, Co, Fe, Ni, and Cu) and other metal ions (including As, Sb and Pb). The binding of metal ions by P₁ inhibits *(i. e.,* reduces or prevents) the production of ROS and/or the accumulation of ROS by these metal ions and/or targets the damage done by ROS that may still be produced by the bound metal ions to the peptide itself. As a result, the damage that can be caused by ROS in the absence of the binding of the metal ions to P₁ is reduced. In particular, P₁ binds Cu(II), Ni(II), Co(II), and Mn(II) with high affinity. It should, therefore, be particularly effective in reducing the damage caused by the production and accumulation of ROS by copper and nickel.

In P₁, Xaa₁ is most preferably Asp, Xaa₂ is most preferably Ala, and Xaa₃ is most preferably Lys. Thus, the preferred sequences of P₁ are Asp Ala His and Asp Ala His Lys [SEQ ID NO:1]. Most preferably the sequence of P₁ is Asp Ala His Lys [SEQ ID NO:1]. Asp Ala His is the minimum sequence ofthe N-terminal metal-binding site of human serum albumin necessary for the high-affinity binding of Cu(II) and Ni(II), and Lys has been reported to contribute to the binding of these metal ions to this site. Other sequences for P₁ may be preferred for use in animals other than humans.

P₂ is (Xaa₄)ₙ, wherein Xaa₄ is any amino acid and n is 0-100. When n is large about 20), the peptides will reduce the damage done by ROS extracellularly. Smaller peptides are better able to enter cells, and smaller peptides can, therefore, be used to reduce the damage done by ROS both intracellularly and extracellularly. Smaller peptides are also less subject to proteolysis. Therefore, in P₂, preferably n is 0-10, more preferably n is 0-5, and most preferably n is 0. Although P₂ may have any sequence, P₂ preferably comprises a sequence which (1) binds a transition metal, (2) is hydrophobic to enhance the ability of the peptide to penetrate cell membranes and/or reach target tissues (e.g., to be able to cross the blood brain barrier), or (3) otherwise stabilizes or enhances the performance of the peptide. P₂ together with P₁ may also be the N-terminal sequence of a protein having an N-terminal metal-binding site with high affinity for copper and nickel, such as human, rat or bovine serum albumin. In the case where n = 100, the peptide would have the sequence of approximately domain 1 of these albumins.

The sequences of many peptides which comprise a binding site for transition metal ions are known. See, e.g., U.S. Patents Nos. 4,022,888, 4,461,724, 4,665,054, 4,760,051, 4,767,753, 4,810,693, 4,877,770, 5,023,237, 5,059,588, 5,102,990, 5,118,665, 5,120,831, 5,135,913, 5,145,838, 5,164,367, 5,591,711, 5,177,061, 5,214,032, 5,252,559, 5,348,943, 5,443,816, 5,538,945, 5,550,183, 5,591,711, 5,690,905, 5,759,515, 5,861,139, 5,891,418, 5,928,955, and 6,017,888, PCT applications WO 94/26295, WO 99/57262 and WO 99/67284, European Patent application 327263, Lappin et al., Inorg. Chem., 17, 1630-34 (1978), Bossu et al., Inorg. Chem., 17, 1634-40 (1978), Chakrabarti, Protein Eng., 4, 57-63 (1990), Adman, Advances In Protein Chemistry, 42, 145-97 (1991), Cotelle et al., J. Inorg. Biochem., 46, 7-15 (1992), Canters et al., FEBS, 325, 39-48 (1993), Regan, Annu. Rev. Biophys. Biomol. Struct., 22, 257-281 (1993), Ueda et al., J. Inorg. Biochem., 55, 123-30 (1994), Ueda et al., Free Radical Biol. Med., 18, 929-33 (1995), Regan, TIBS, 20, 280-85 (1995), Ueda et al., Chem. Pharm. Bull., 43, 359-61 (1995), Bal et al., Chem. Res. Toxicol., 10, 906-914 (1997), Bal et al., Chem. Res. Toxicol., 10, 915-21 (1997), Koch et al., Chem. Biol., 4, 549-60 (1997), Kowalik-Jankowska et al., J. Inorg. Biochem., 66,193-96 (1997), Harford and Sarkar, Acc. Chem. Res., 30,123-130 (1997), Prince et al., TIBS, 23, 197-98 (1998), Mlynarz, et al., *Speciation 98: Abstracts,* http://www.iate.u-szeged.hu/∼spec98/abstr/mlynar.html, and Aitken, Molec. Biotechnol.,12, 241-53 (1999), Whittal et al., Protein Science, 9, 332-343 (2000). P₂ may comprise the sequence of one or more of the metal-binding sites of these peptides.

When P₂ comprises a metal-binding site, it preferably has a sequence which includes a short spacer sequence between P₁ and the metal binding site of P₂, so that the metal-binding sites of P₁and P₂ may potentially cooperatively bind metal ions (similar to a 2:1 peptide:metal complex; see Example 10). Preferably, the spacer sequence is composed of 1-5, preferably 1-3, neutral amino acids. Thus, the spacer sequence may be Gly, Gly Gly, Gly Ala Gly, Pro, Gly Pro Gly, etc.

In particular, when P₂ comprises a metal-binding site, it preferably comprises one of the following sequences: (Xaa₄)ₘ Xaa₃ His Xaa₂ Xaa₅, (Xaa₄)ₘ His Xaa₂ Xaa₅, (Xaa₄)ₘ Xaa₅ Xaa₂ His Xaa_{3,} or (Xaa₄)ₘ Xaa₅ Xaa₂ His. Xaa₂, Xaa₃ and Xaa₄ are defined above, and m is 0-5, preferably 1-3. The Xaa₄ amino acids form a short spacer sequence between P₁ and the metal binding site of P₂ so that the metal-binding sites of P₁ and P₂ may cooperatively bind metal ions, and Xaa₄ is preferably a neutral amino acid (see the previous paragraph). Xaa₅ is an amino acid having a free side-chain -NH₂, preferably Om or Lys, more preferably Orn. See Harford and Sarkar, Acc. Chem. Res., 30, 123-130 (1997) (the free side-chain -NH₂ of Orn within a peptide sequence has been reported to successfully substitute for the free N-terminal -NH₂ of the ATCUN motif). Thus, for instance, P, - P₂ could be Asp Ala His Gly Gly His Ala Om [SEQ ID NO:2].

The amino acids of the peptide may be L-amino acids, D-amino acids, or a combination thereof. Preferably, at least one of the amino acids of P₁ is a D-amino acid (preferably Xaa₁ and/or His), except for β-Ala. Most preferably, all ofthe amino acids of P₁, other than β-Ala, are D-amino acids. Also, preferably about 50% of the amino acids of P₂ are D-amino acids, and most preferably all of the amino acids of P₂ are D-amino acids. D-amino acids are preferred because peptides containing D-amino acids are resistant to proteolytic enzymes, such as those that would be encountered upon administration of the peptide to an animal (including humans) or would be present in an excised organ perfused with a solution containing the peptide. Also, the use of D-amino acids would not alter the ability of the peptide to bind metal ions, including the ability of the peptide to bind copper with high affinity.

The peptides of the invention may be made by methods well known in the art. For instance, the peptides, whether containing L-amino acids, D-amino acids, or a combination of L- and D-amino acids, may be synthesized by standard solid-phase peptide synthesis methods. Suitable techniques are well known in the art, and include those described in Merrifield, in Chem. Polypeptides, pp. 335-61 (Katsoyannis and Panayotis eds. 1973); Merrifield, J. Am. Chem. Soc. 85, 2149 (1963); Davis et al., Biochem. Int'1, 10, 394-414 (1985); Stewart and Young, Solid Phase Peptide Synthesis (1969); U.S. Patents Nos. 3, 941,763 and 5,786,335; Finn et al., in The Proteins, 3rd ed., vol. 2, pp.105-253 (1976); and Erickson et al. in The Proteins, 3rd ed., vol. 2, pp. 257-527 (1976). See also, Polish Patent 315474 (synthesis ofHMS-containing peptides). Alternatively, the peptides may be synthesized by recombinant DNA techniques if they contain only L-amino acids. Recombinant DNA methods and suitable host cells, vectors and other reagents for use therein, are well known in the art. See, *e.g.,* Maniatis et al., Molecular Cloning: A Laboratory Manual, Cold Spring Harbor, NY (1982), Sambrook et al., Molecular Cloning: A Laboratory Manual, Cold Spring Harbor, NY (1989).

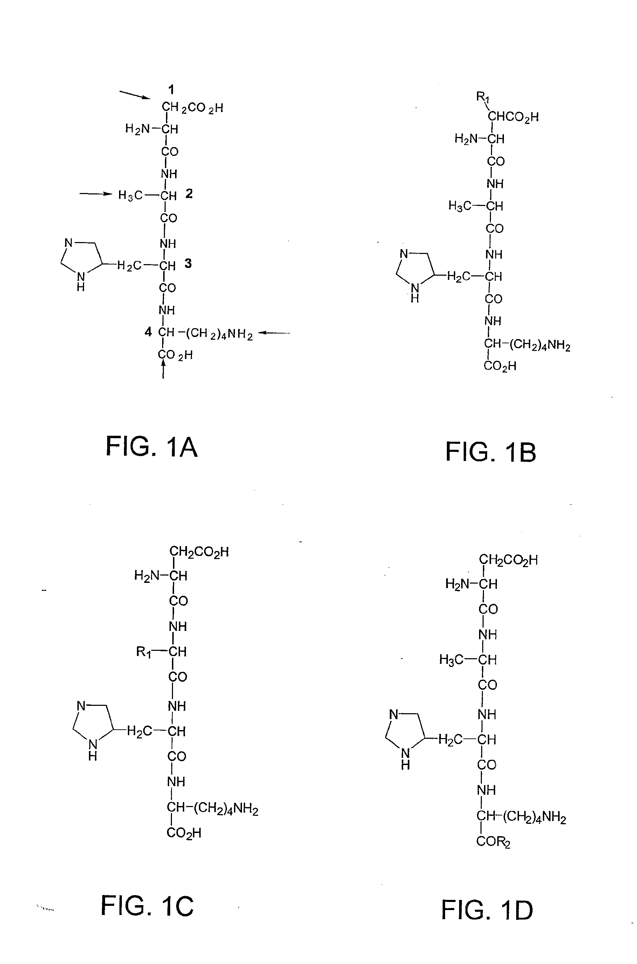

The invention further comprises derivatives of the peptide P₁ - P₂, whether composed ofL-amino acids, D-amino acids, or a combination ofL- and D-amino acids, which are more resistant to proteolytic enzymes, more lipid soluble (to allow the peptides to more readily penetrate cell membranes and/or reach target organs, such as the brain), or both. As illustrated in Figure 1A, P₁ can be modified in the regions indicated by the arrows without altering the metal binding function of P₁. In particular, P₁ can be substituted at carbons 1 or 2 with R₁, and the terminal -COOH of P₁ can be substituted with protecting group R₂ (Figures 1B-D). P₂ can be modified in ways similar to those described for P₁ to make P₂ more resistant to proteolytic enzymes, more lipid soluble, or both.

R₁ can be a straight-chain or branched-chain alkyl containing from 1 to 16 carbon atoms, and the term "alkyl" includes the R and S isomers. R₁ can also be an aryl or heteroaryl containing 1 or 2 rings. The term "aryl" means a compound containing at least one aromatic ring (e.g., phenyl, naphthyl, and diphenyl). The term "heteroaryl" means an aryl wherein at least one of the rings contains one or more atoms of S, N or O. These substitutions do not substantially decrease the ability of P₁ to bind metal ions. In particular, the ability of P₁ to bind copper with high affinity is not decreased by these substitutions. For instance, some of the substituents, such as a n-butyl attached to carbon 2 (see Figure 1C, R₁ is n-butyl) should increase the affinity of the peptide for metal ions, such as copper, due to the inductive effect of the alkyl group. Substitution of carbon 2 (Figure 1C) with an aryl, heteroaryl, or a long chain alkyl (about 6-16 carbon atoms) should enhance transport of the peptide across lipid membranes.

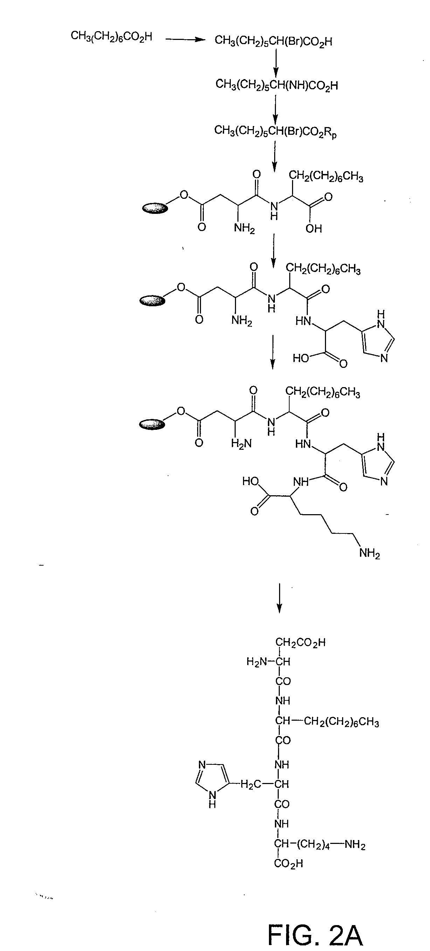

As noted above, methods of synthesizing peptides by solid phase synthesis are well known. These methods can be modified to prepare the derivatives shown in Figures 1B-C. For example, the derivative of P₁ illustrated in Figure 1C, wherein R₁ is octyl, can be prepared as illustrated in Figure 2A. In Figure 2A, the elliptical element represents the polymer resin and Rₚ is a standard carboxyl protecting group. As illustrated in Figure 2A, octanoic acid (freshly distilled) is treated with dry bromine followed by phosphorus trichloride. The mixture is heated to about 100°C and kept at that temperature for 4 hours. α-Bromooctanoic acid is obtained as a colorless liquid upon distillation. Amination of the bromoacid is achieved by allowing the acid and an ammonia solution to stand at 40-50° C for 30 hours. The octyl derivative of the amino acid is obtained by removing ammonium bromide with methanol washes. Classical resolution methods give the desired optically-pure D-form. Other derivatives wherein R₁ is an alkyl, aryl or heteroaryl can be prepared in the manner illustrated in Figure 2A.

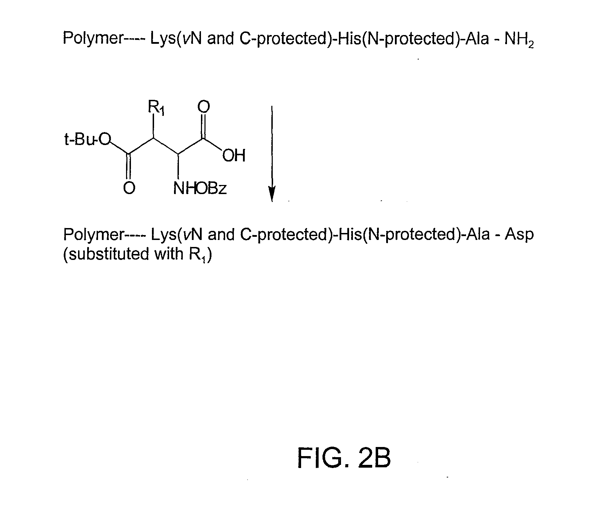

In addition, the derivative of P₁ illustrated in Figure 1B, wherein R₁ is phenyl, can be prepared as illustrated in Figure 2B. In Figure 2B, Polymer is the resin, t-Bu is t-butyl, and Bz is benzyl. Other derivatives wherein R₁ is an alkyl, aryl or heteroaryl can be prepared in the manner illustrated in Figure 2B.

R₂ can be -NH ₂, -NHR₁, -N(R₁)₂, -OR₁, or R₁ (see Figure 1D), wherein R₁ is defined above. These derivatives can be prepared as the last step of a solid-phase peptide synthesis before the peptide is removed from the resin by methods well known in the art. Substitutions with R₂ do not substantially decrease the ability of P₁ to bind metal ions.

In addition, P₁ and P₂ can be substituted with non-peptide functional groups that bind metal ions. These metal-binding functional groups can be attached to one or more pendent groups of the peptide, and the resulting peptide derivatives will possess one or more sites that are capable of binding metal ions, in addition to the binding site provided by P₁ and, optionally, the binding site provided by P₂. As a consequence, the ability of such peptide derivatives to bind metal ions is improved as compared to the corresponding unmodified peptide. For instance, the peptide derivative can bind two of the same type of metal ion instead of one (e.g., two Cu(II)), the peptide derivative can bind two different metal ions instead of one type of metal ion *(e.g.,* one Cu(II) and one Fe(III)), or the peptide derivative can bind one metal ion better (e.g., with greater affinity) than the corresponding unmodified peptide.

Metal-binding functional groups include polyamines (e.g., diamines, triamines, etc.). Suitable diamines include 1,2-alkyldiamines, preferably alkyl diamines wherein the alkyl contains 2-10 carbon atoms (e.g., H₂N - (CH₂)ₙ - NH₂, wherein n = 2-10). Suitable diamines also include 1,2-aryldiamines, preferably benzene diamines (e.g., 1,2-diaminobenzene). Suitable diamines further include 1,2-cyclic alkane diamines. "Cyclic alkanes" are compounds containing 1-3 rings, each containing 5-7 carbon atoms. Preferably the cyclic alkane diamine is 1,2-diaminocylcohexane (cyclohexane diamine).

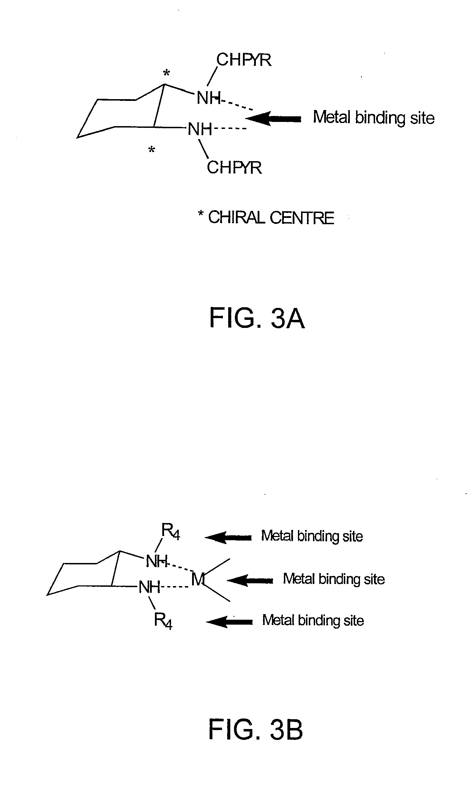

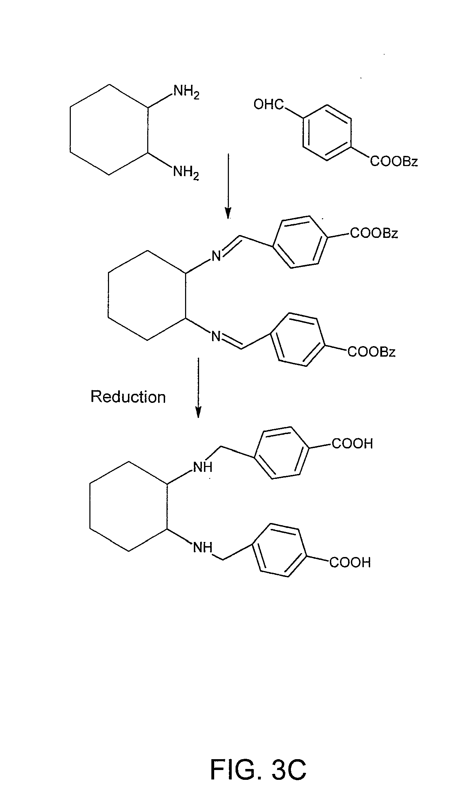

A particularly preferred diamine is 1,2-diaminocyclohexane (Figures 3A-B). Previous studies carried out by Rao & P. Williams (J. ChromatographyA, 693, 633 (1995)) have shown that a cyclohexane diamine derivative (Figure 3A, where PYR is pyridine) binds to a variety of metal ions. The resulting metal chelator has been successfully used to resolve amino acids and peptides, showing that the molecule has a very high affinity for α-amino acids, forming a very stable coordination complex, which is unique in many respects. 1,2-Diaminocyclohexane possesses a reactive amino functional group to which a peptide of the invention can be attached. See Figure 3B, where M is a metal ion and at least one R₄ is -alkyl-CO-peptide, -aryl-CO-peptide, -aryl-alkyl-CO-peptide, or -allcyl-aryl-CO-peptide (see also Figures 3C-D). The other R₄ may be the same or may be -alkyl-COOH, -aryl-COOH, -aryl-alkyl-COOH, or alkyl-aryl-COOH. Derivatives of the type shown in Figure 3B will have several metal-binding sites and can, therefore, be expected to bind metal ions more readily than the unsubstituted peptide. Further, due to the presence of the cyclohexane functionality, the compound will possess lipid-like characteristic which will aid its transport across lipid membranes.

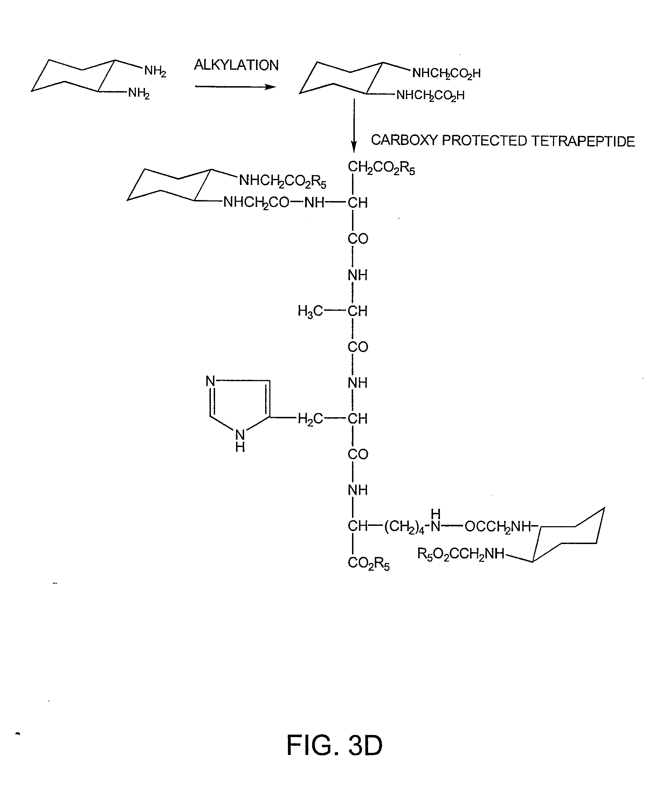

Cyclohexane diamine derivatives of the peptides of the invention can be prepared by two distinct routes. The first involves initial condensation with an aldehyde followed by reduction (see Figure 3C; in Figure 3C Bz is benzyl). A number of aldehydes (alkyl and aryl) react readily with cyclohexane diamine at room temperature, forming an oxime. The oxime can be reduced with sodium borohydride under anaerobic conditions to give the diacid derivative. The carboxyl moieties are then reacted with the free amino groups present in carboxy-protected P₁ to give the cyclohexane diamine derivative of the peptide. The second route is a direct alkylation process which is illustrated in Figure 3D. For example, cyclohexane diamine is treated with bromoacetic acid to give the diacetic acid derivative. The carboxyl moieties are then reacted with the free amino groups present in carboxy-protected P₁ to give the derivative. In Figure 3D, R₅ is H or another peptide. When R₅ is H, the derivative can be further reacted to produce typical carboxylic acid derivatives, such as esters, by methods well known in the art. Metal binding experiments have indicated that the presence or absence of this group does not have a bearing on the metal binding capacity of the whole molecule. However, these groups would either make the molecule hydrophobic or hydrophilic, depending upon the substituent, and this may, in turn, have an effect on delivery of the molecule across membranes or to target tissues. These two synthetic routes will work for the synthesis of diamine peptide derivatives using the other diamines described above.

Additional suitable polyamines and polyamine derivatives and methods of attaching them to peptides are described in U.S. Patents Nos. 5,101,041 and 5,650,134. Other polyamine chelators suitable for attachment to peptides are known. See, e.g., U.S. Patents Nos. 5,422,096, 5,527,522, 5,628,982, 5,874,573, and 5,906,996 and PCT applications WO 97/44313, WO 97/49409, and WO 99/39706.

It is well known that vicinal diacids bind to metal ions, and the affinity for copper is particularly high. It is therefore envisaged that a peptide having a vicinal diacid functional group will be extremely effective in metal binding. Suitable vicinal diacids include any 1,2-alkyldiacid, such as diacetic acid (succinic acid), and any 1,2-aryldiacid.

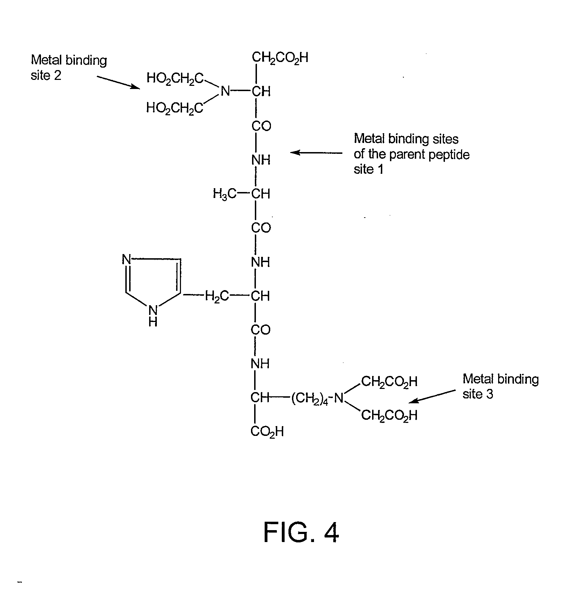

The amino groups of the peptide can be reacted with diacetic acid to produce a diacid derivative (see Figure 4). This can be conveniently accomplished by reacting the amino groups of the resin-bound peptide with a halogenated acetic acid (e.g., bromoacetic acid or chloroacetic acid) or a halogenated acetic acid derivative (e.g., benzyloxy ester). Solid phase synthetic procedures enable removal ofunreacted materials by washing with solvent. The final product is released from the resin by hydrolytic cleavage. Other diacid derivatives of the peptides of the invention can be made in the same manner.

Polyaminopolycarboxylic acids are known to bind metals, such as copper and iron. Suitable polyaminopolycarboxylic acids for making derivatives ofthe peptides ofthe invention and methods of attaching them to peptides are described in U.S. Patents Nos. 5,807,535 and 5,650,134, and PCT application WO 93/23425. See also, U.S. Patent No. 5,739,395.

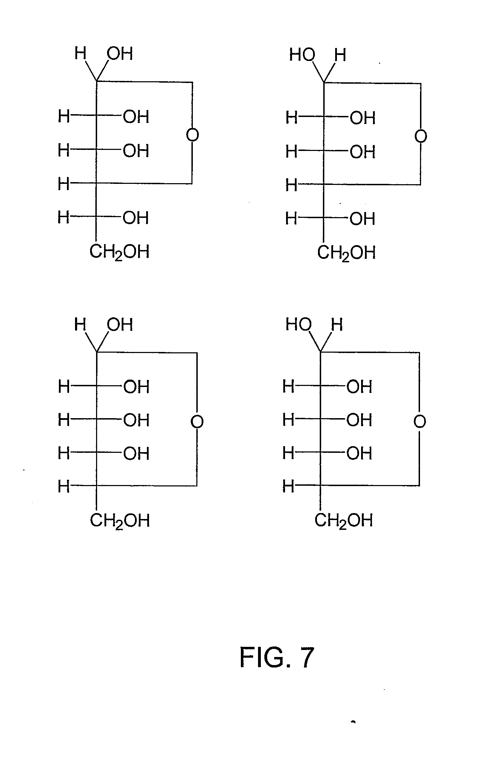

Vicinal polyhydroxyl derivatives are also included in the invention. Suitable vicinal polyhydroxyls include monosaccharides and polysaccharides (i. e., disaccharide, trisaccharide, etc.). Presently preferred are monosaccharides. See Figure 7. The monosaccharides fall into two major categories - furanoses and pyranoses. One of the prime examples of a furanose ring system is glucose. The hydroxyl groups of glucose can be protected as benzyl or labile t-butyloxy functional groups, while leaving the aldehyde free to react with an amine group (e.g., that of lysine) of the tetrapeptide. Mild reduction/hydrolysis produces the monosaccharide peptide derivative. Other monosaccharide peptide derivatives can be prepared in this manner.

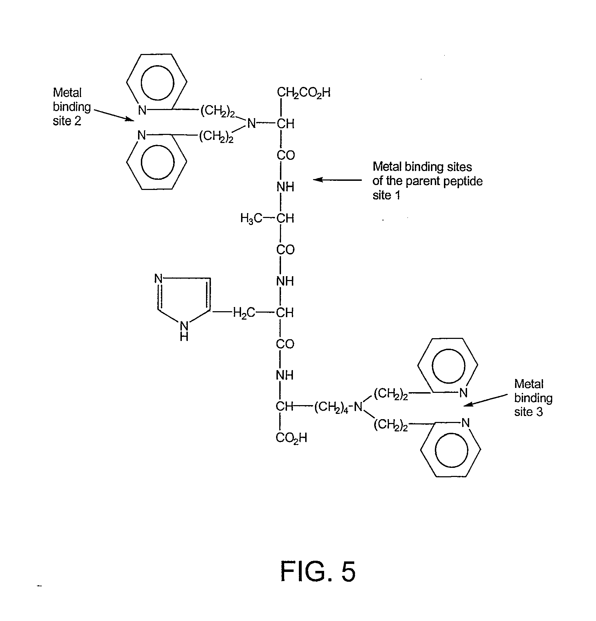

Bispyridylethylamine derivatives are known to form strong complexes with divalent metal ions. When attached to the peptide, this functional group would provide additional chelating sites for metal ions, including copper. The bispyridylethyl derivative of the tetrapeptide Asp Ala His Lys [SEQ ID NO:1] is shown in Figure 5. It is anticipated that the metal-binding capacity of this tetrapeptide derivative will be increased by at least three-fold as compared to the underivatized peptide. The preparation of this bispyridylethylamine derivative shares some similarities with the synthesis of diacid derivatives. The two amino groups of the tetrapeptide (one at Asp and the other at Lys) are reacted with 2-bromoethylpyridine to give the tetra-substituted peptide derivative. The reaction is accomplished by reacting the resin-bound tetrapeptide with the bromoethylpyridine, followed by cleavage of the product from the resin.

Phenanthroline is another heterocyclic compound capable of binding divalent metal ions. Phenanthroline derivatives of the peptides can be synthesized in the same manner as for the bispyridylethylamine derivatives.

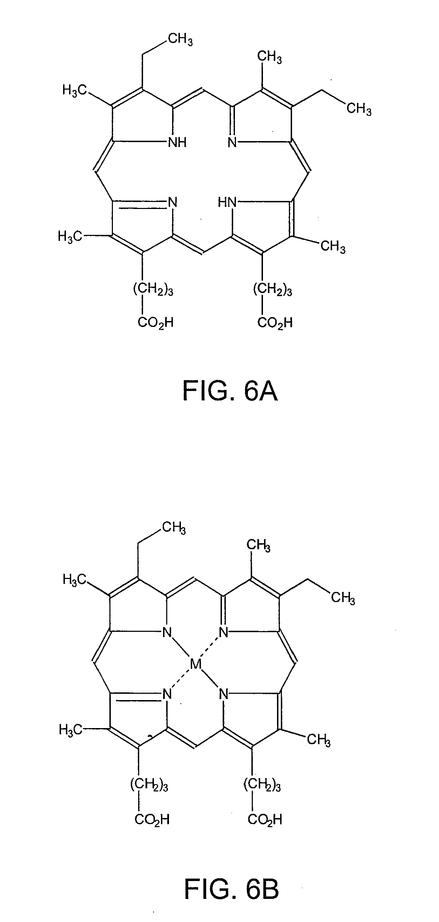

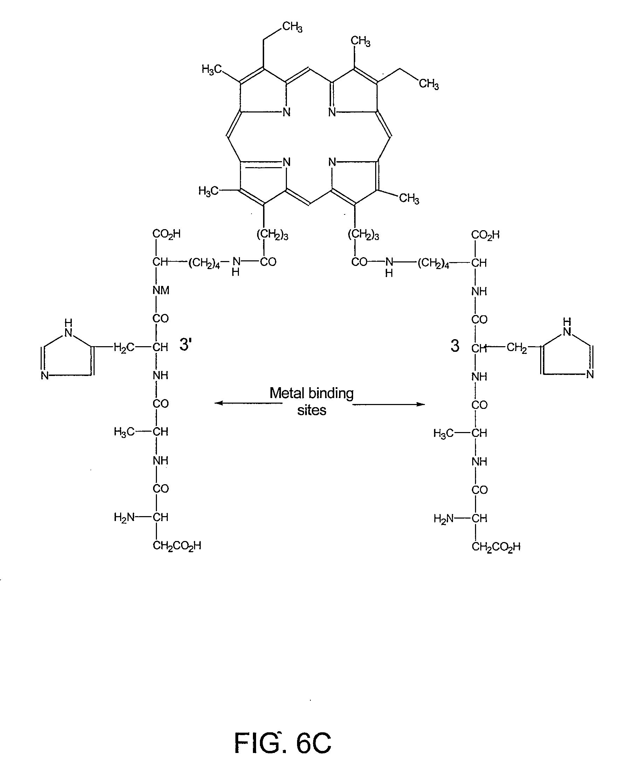

Porphyrins are a group of compounds found in all living matter and contain a tetrapyrrolic macrocycle capable of binding to metals. Heme, chlorophyll and corrins are prime examples of this class of compounds containing iron, magnesium and cobalt, respectively. Mesoporphyrin IX (Figure 6A-B, where M is a metal ion) is derived from heme and has been observed to possess specific affinity for copper. Addition of this structure to a peptide of the invention would produce a porphyrin-peptide derivative possessing several sites for binding of copper (see Figure 6C). In addition to their roles in metal binding, the imidazole residues at positions 3 and 3' of the tetrapeptide shown in Figure 6C may provide a binding site for metals other than copper, thereby stabilizing the porphyrin-metal complex. In particular, cyanocobalamine (vitamin B-12) contains cobalt as the metal in the porphyrin nucleus, and the complex is stabilized by the imidazole groups. On the basis of this analogy it is anticipated that the porphyrin-tetrapeptide derivative would bind cobalt (or other metals) at normal physiological conditions in the prophyrin nucleus and that the complex would be stabilized by the His imidazole groups.

To prepare the porphyrin-peptide derivative shown in Figure 6C, the carboxyl groups of mesoporphyrin IX can be activated and coupled with the amino groups of the peptide employing standard solid-phase peptide synthesis. Typically, the free amino group of the lysine residue of the resin-bound peptide can be coupled with carboxy activated porphyrin nucleus. The condensation product can be cleaved off the resin using standard methods. This method can be used to synthesize other porphyrin derivatives of peptides of the invention.

Other suitable porphyrins and macrocyclic chelators and methods of attaching them to peptides are described in U.S. Patents Nos. 5,994,339 and 5,087,696. Other porphyrins and macrocyclic chelators that could be attached to peptides are known. See, e.g., U. S. Patents Nos. 5,422,096, 5,527,522, 5,628,982, 5,637,311, 5,874,573, and 6,004,953, PCT applications WO 97/44313 and WO 99/39706.

A variety of additional metal chelators and methods of attaching them to proteins are described in U.S. Patent No. 5,683,907.

Dithiocarbamates are known to bind metals, including iron. Suitable dithiocarbamates for making derivatives of the peptides of the invention are described in U.S. Patents Nos. 5,380,747 and 5,922,761.

Hydroxypyridones are also known to be iron chelators. Suitable hydroxypyridones for making derivatives of the peptides of the invention are described in U.S. Patents Nos. 4,912,118 and 5,104,865 and PCT application WO 98/54138.

Additional non-peptide metal chelators are known in the art or will be developed. Methods of attaching chemical compounds to proteins and peptides are well known in the art, and attaching non-peptide metal chelators to the peptides of the invention is within the skill in the art. See, e.g., those patents cited above describing such attachment methods.

As can be appreciated, the non-peptide, metal-binding functional groups could be attached to another metal-binding peptide (MBP) in the same manner as they are to peptide P₁ - P₂. The resulting peptide derivatives would contain one or more metal-binding functional groups in addition to the metal-binding site of MBP. Preferably, MBP contains from 2-10, more preferably 3-5, amino acids. Preferably MBP contains one or more D-amino acids; most preferably all of the amino acids ofMBP are D-amino acids. As described above, the sequences of many metal-binding peptides are known. These peptides and peptides comprising the metal-binding sites of these peptides can be prepared in the same ways as described above for peptide P₁ - P₂. Derivatives of these peptides having one or more metal-binding functional group attached to the peptide can be prepared in the same ways as described above for derivatives of peptide P₁ - P₂.

The invention also provides metal-binding peptide dimers of the formula:

P₃-L-P₃

P₃ is any peptide capable of binding a metal ion, and each P₃ may be the same or different. Each P₃ preferably contains 2-10, more preferably 3-5, amino acids. As described above, metal-binding peptides are known, and each P₃ may comprise the sequence of one or more of the metal-binding sites of these peptides. Although each P₃ may be substituted as described above for P₁ and P₂, including with a non-peptide, metal-binding functional group (see particularly Figure 6C), both P₃ peptides are preferably unsubstituted. P₃ may also comprise any amino acid sequence substituted with a non-peptide, metal-binding functional group as described above to provide the metal-binding capability of P₃. Preferably, each P₃ is an unsubstituted metal-binding peptide (*i.e.,* an unsubstituted peptide comprising a peptide sequence which binds metal ions). Most preferably, one or both of the P₃ groups is P₁ (*i.e.,* the dimers have the sequence P₃ - S - P₁, P₁ - S - P₃ or, most preferably, P₁ - S - P₁). P₁ is defined above.

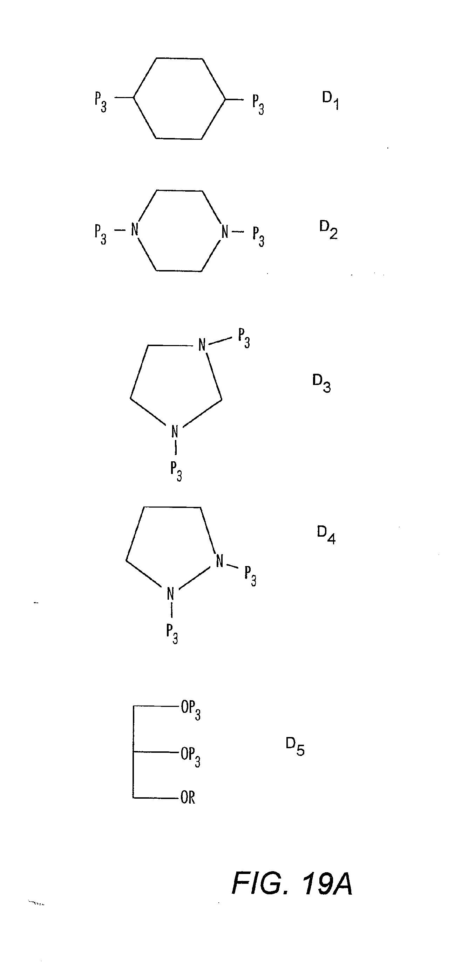

L is a linker which is attached to the C-terminal amino acid of each P₃. L may be any chemical group which can connect the two P₃ peptides through their C-terminal amino acids which is physiologically-acceptable. By "physiologically-acceptable" is meant that a peptide dimer containing the linker L is not toxic to an animal (including a human) or an organ to which the peptide dimer is administered as a result of the inclusion of the linker L in the peptide dimer. Preferably, L links the two P₃ groups so that they can cooperatively bind metal ions (similar to a 2:1 peptide:metal complex; see Example 10). L is also preferably neutral. Most preferably, L is a straight-chain or branched-chain alkane or alkene residue containing from 1-18, preferably from 2-8, carbon atoms (*e.g.,* -CH₂-, -CH₂CH₂-, -CH₂CH₂CH-, -CH₂CH₂(CH₃)CH₂-, -CHCH-, etc.) or a cyclic alkane or alkene residue containing from 3-8, preferably from 5-6, carbon atoms (see Figure 19A, compound D₁), preferably attached to a P₃ by means of an amide linkage. Such linkers are particularly preferred because they impart hydrophobicity to the peptide dimers. In another preferred embodiment, L is a nitrogen-containing heterocyclic alkane residue (see Figure 19A, compounds D₂, D₃ and D₄), preferably a piperazide (see Figure 19A, compound D₂). In another preferred embodiment L is a glyceryl ester (see Figure 19A, compound D₅; in formula D₅, R is an alkyl or aryl containing, preferably containing 1-6 carbon atoms). These preferred linkers L will allow the two peptides P₃ to bind metal ions cooperatively and are biocompatible, and the peptide dimers can be made easily and in large quantities. By "biocompatible" is meant that a peptide dimer containing the linker L does not produce any undesirable side-effects due to the linker L in an animal (including a human) or an organ to which the peptide dimer is administered.

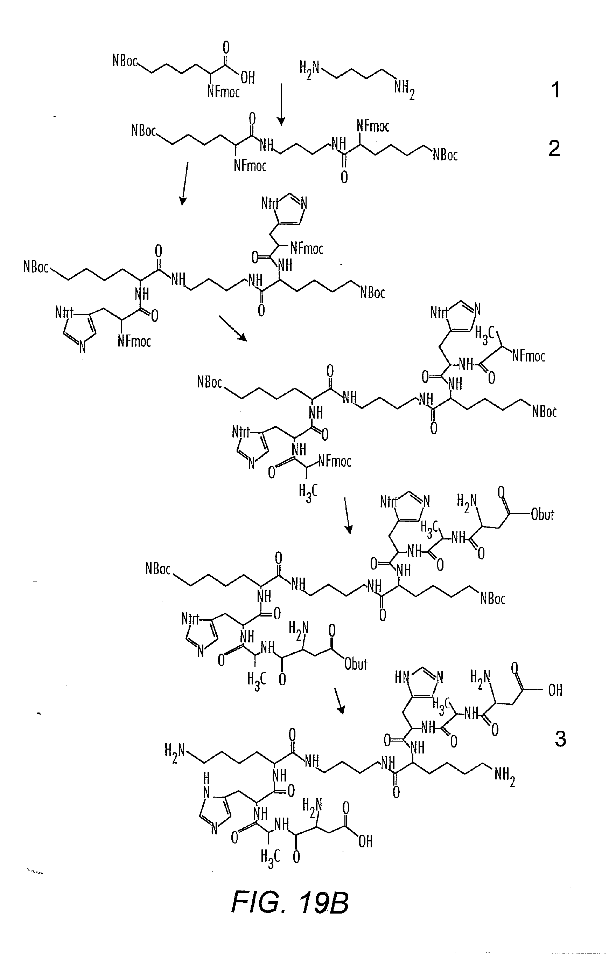

Methods of synthesizing the peptide dimers are illustrated in Figures 19B-D. In general, the C-terminal amino acids (protected by methods and protecting groups well known in the art) of the two P₃ groups are attached to L, and the resulting amino acid dimers used in standard peptide synthetic methods to make the peptide dimers.

For instance, a peptide dimer, where each peptide has the sequence Asp Ala His Lys, can be synthesized by coupling protected lysines to a free diamine functional group, either as an acid chloride or by using standard coupling agents used in peptide synthesis (see Figures 19B-C). Many suitable diamines are available commercially or suitable diamines can be readily synthesized by methods known in the art.

For instance, the lysine dimer **2** (Figure 19B) can be prepared as follows. To a stirred solution of9-fluorenylmethyloxycarbonyl (Fmoc)- and t-benzyloxycarbonyl(Boc)- protected D-Lys (Fmoc-D-Lys(Boc)-OH) (20 mmole) in dry dimethylformamide (DMF;100 mL; dry argon flushed) are added butane-1,4-diamine **1** and 2-(1H-benzotriazole-1-yl)-1,2,3,3-tetramethyluroniumtetrafluoroborate (TBTU; 0.5 mmole). The solution is stirred for 36 hours at room temperature. The bis-protected lysine **2** is isolated by flash chromatography over silica and elution with mixtures of ethyl acetate/methanol. The peptide dimer **3** is then prepared from the protected lysine dimer **2** employing classical peptide synthesis methodology (see Figure 19B).

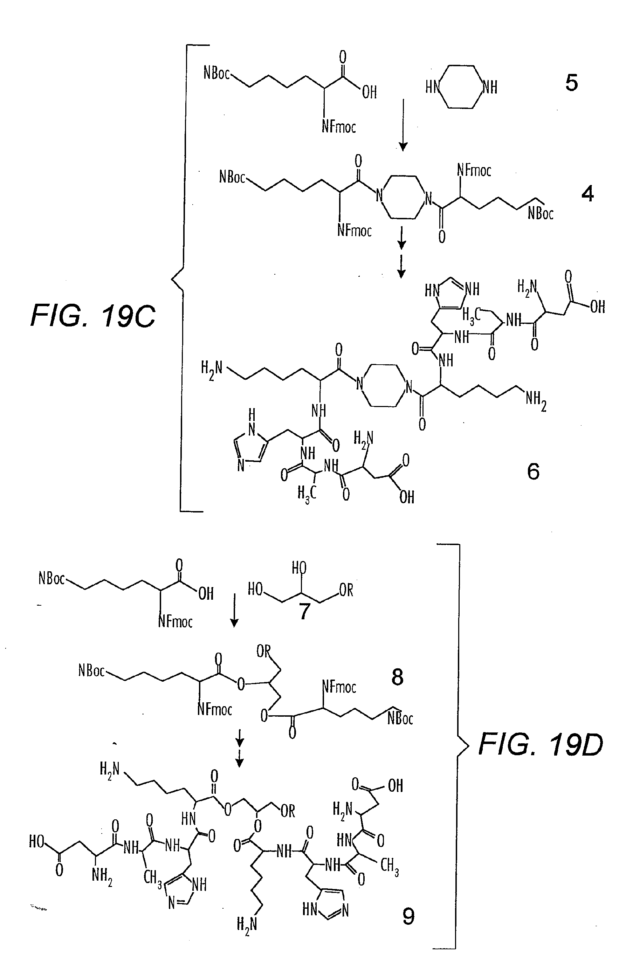

Another peptide dimer, where each peptide has the sequence Asp Ala His Lys, can be synthesized as follows. First, a different protected lysine dimer **4** is synthesized by acylating the two amino centers of a piperazine **5** (see Figure 19C; see also Chambrier et al., Proc. Natl. Acad. Sci., 96, 10824-10829 (1999)). Then, the remainder of the amino acid residues are added employing standard peptide synthesis methodology to give the peptide dimer **6** (see Figure 19C).

Peptide dimers, where each peptide has the sequence Asp Ala His Lys and where L is a glyceryl ester, can be synthesized as follows. The 3-substituted propane-1,2-diols of formula **7** in Figure 19D, wherein R is an alkyl or aryl, are commercially available. A lysine diester **8,** wherein R is methyl, can be prepared as follows (see Figure 19D). To a stirred solution of Fmoc-D-Lys(Boc)-OH (20 mmole) in dry toluene (100 mL; dry argon flushed) is added 3-methoxypropane-1,2-diol (200 mmole) and imidazole (15 mmole). The solution is stirred for 36 hours at room temperature. The solvent is removed in vacuo, and the residue is dissolved in ethyl acetate. This solution is washed with citric acid solution (2%), water, 0.5 N NaHCO₃ solution, and again with water; then the organic layer is dried over magnesium sulphate (removal of the solvent gives a pale yellow residue). The bis-protected lysine **8** is isolated by flash chromatography over silica and elution with mixtures of ethyl acetate/methanol. The peptide dimer **9** is then prepared from the protected lysine dimer **8** employing classical peptide synthesis methodology (see Figure 19D).

The physiologically-acceptable salts ofthe metal-binding compounds are also included in the invention. Physiologically-acceptable salts include conventional non-toxic salts, such as salts derived from inorganic acids (such as hydrochloric, hydrobromic, sulfuric, phosphoric, nitric, and the like), organic acids (such as acetic, propionic, succinic, glycolic, stearic, lactic, malic, tartaric, citric, glutamic, benzoic, salicylic, and the like) or bases (such as the hydroxide, carbonate or bicarbonate of a pharmaceutically-acceptable metal cation). The salts are prepared in a conventional manner, e.g., by neutralizing the free base form of the compound with an acid.

A metal-binding compound ofthe invention can be used to reduce the damage done by ROS or to reduce excess metal ion concentrations in an animal. To do so, a metal-binding compound of the invention is administered to the animal. Preferably, the animal is a mammal, such as a rabbit, goat, dog, cat, horse or human. Effective dosage forms, modes of administration and dosage amounts for the various compounds of the invention may be determined empirically, and making such determinations is within the skill of the art. It has been found that an effective dosage is from about 2 to about 200 mg/kg, preferably from about 10 to about 40 mg/kg, most preferably about 20 mg/kg. However, it is understood by those skilled in the art that the dosage amount will vary with the particular metal-binding compound employed, the disease or condition to be treated, the severity of the disease or condition, the route(s) of administration, the rate of excretion of the compound, the duration of the treatment, the identify of any other drugs being administered to the animal, the age, size and species of the animal, and like factors known in the medical and veterinary arts. In general, a suitable daily dose of a compound of the present invention will be that amount of the compound which is the lowest dose effective to produce a therapeutic effect. However, the daily dosage will be determined by an attending physician or veterinarian within the scope of sound medical judgment. If desired, the effective daily dose may be administered as two, three, four, five, six or more sub-doses, administered separately at appropriate intervals throughout the day. Administration of the compound should be continued until an acceptable response is achieved.

The compounds of the present invention may be administered to an animal patient for therapy by any suitable route of administration, including orally, nasally, rectally, vaginally, parenterally (e.g., intravenously, intraspinally, intraperitoneally, subcutaneously, or intramuscularly), intracistemally, transdermally, intracranially, intracerebrally, and topically (including buccally and sublingually). The preferred routes of administration are orally and intravenously.

While it is possible for a metal-binding compound ofthe present invention to be administered alone, it is preferable to administer the compound as a pharmaceutical formulation (composition). The pharmaceutical compositions of the invention comprise a metal-binding compound or compounds of the invention as an active ingredient in admixture with one or more pharmaceutically-acceptable carriers and, optionally, with one or more other compounds, drugs or other materials. Each carrier must be "acceptable" in the sense of being compatible with the other ingredients of the formulation and not injurious to the animal. Pharmaceutically-acceptable carriers are well known in the art. Regardless of the route of administration selected, the compounds of the present invention are formulated into pharmaceutically-acceptable dosage forms by conventional methods known to those of skill in the art. See, *e.g., Remington's Pharmaceutical Sciences.*

Formulations of the invention suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of a compound of the present invention as an active ingredient. A compound of the present invention may also be administered as bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredient only, or preferentially, in a certain portion of the gastrointestinal tract, optionally, in a delayed manner. Examples of embedding compositions which can be used include polymeric substances and waxes. The active ingredient can also be in microencapsulated form.

Liquid dosage forms for oral administration of the compounds of the invention include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or salicylate, and which is solid at room temperature, but liquid at body temperature and, therefore, will melt in the rectum or vaginal cavity and release the active compound. Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches, drops and inhalants. The active compound may be mixed under sterile conditions with a pharmaceutically-acceptable carrier, and with any buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane.

Transdermal patches have the added advantage of providing controlled delivery of a compound of the invention to the body. Such dosage forms can be made by dissolving, dispersing or otherwise incorporating a compound of the invention in a proper medium, such as an elastomeric matrix material. Absorption enhancers can also be used to increase the flux ofthe compound across the skin. The rate of such flux can be controlled by either providing a rate-controlling membrane or dispersing the compound in a polymer matrix or gel.

Pharmaceutical compositions of this invention suitable for parenteral administrations comprise one or more compounds of the invention in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monosterate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug is accomplished by dissolving or suspending the drug in an oil vehicle.

Injectable depot forms are made by forming microencapsule matrices of the drug in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue. The injectable materials can be sterilized for example, by filtration through a bacterial-retaining filter.

The formulations may be presented in unit-dose ormulti-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

As noted above, ROS have been reported to play a major role in a variety of diseases and conditions. See Manso, Rev. Port. Cardiol., 11, 997-999 (1992); Florence, Aust. NZJ. Opthalmol., 23, 3-7 (1992); Stohs, J. Basic Clin. Physiol. Pharmacol., 6, 205-228 (1995); Knight, Ann. Clin. Lab. Sci., 25, 111-121 (1995); Kerr et al., Heart & Lung, 25, 200-209 (1996). Diseases involving or caused by excess metal ions are also known. The metal-binding compounds of the invention can be used to treat any of these diseases and conditions and other diseases and conditions in which ROS or transition metals play a role. Specific diseases and conditions treatable with the metal-binding compounds of the invention include adult respiratory distress syndrome, aging, AIDS, artherosclerosis (hypertension, senility and impotence), arthritis, asthma, autoimmune diseases, cancer (e.g., kidney, liver, colon, and brain), carcinogenesis, cellular damage caused by ionizing radiation (e.g., radiation of tumors), chronic granulomatous disease, cirrhosis, Crohn's disease, diabetes (diabetic retinopathy, renal disease, impotence and peripheral vascular disease), eye diseases (e.g., cataracts, central artery occlusion, benign monoclonal gammopathy, and macular degeneration), emphysema, inflammation, ischemia, neoplastic diseases, neurological trauma, neurodegenerative diseases (e.g., Alzheimer's disease, amyotropic lateral sclerosis, Parkinson's disease, multiple sclerosis, and senile dementia), pancreatitis, peripheral vascular disease, pulmonary embolism, renal disease (dialysis patients), reperfusion, shock, tissue damage occurring upon administration of chemotherapeutics, tissue damage after surgery *(e.g.,* transplantation surgery, open heart surgery, and any surgery where the blood supply to a tissue is cut off, and surgical ischemia ofthe limbs (tourniquet injury)), toxic reactions (e.g, herbicide poisoning, transition metal (copper, cobalt, and nickel) poisoning, carbon monoxide poisoning, and antibiotic toxicity), traumatic crush injury, and Wilson's disease (congenital high levels of copper). Specific ischemic conditions and diseases treatable with the metal-binding compounds of the invention include:
Central nervous system ischemia -
   Brain ischemia after surgery
   Hyperthermia brain injury
   Perinatal hypoxia-induced ischemia ("cerebral palsy")
   Spinal cord injury
   Stroke (thrombotic, embolic or hemorrhagic cerebrovascular accident)
   Transient ischemic attack
   Traumatic brain injury
Cardiac ischemia -
   Acute myocardial infarction
   Angina pectoris
   Arrythmias
   Cardiac ischemia after surgery
   Congestive heart failure
   Myocardial "stunning"
Ischemic bowel disease
Placental ischemia
Pulmonary embolism
Surgery where the blood supply to a tissue or organ is cut off -
   Angioplasty
   Cardiac bypass surgery
   Transplantation surgery (both the donor organ and the recipient of the organ) Surgical ischemia of the limbs (tourniquet injury).

A compound of the invention is preferably administered prophylactically. For instance, a compound of the invention is preferably administered prior to and/or simultaneously with reperfusion of an ischemic tissue or organ (e.g., prior to and/or simultaneously with angioplasty or treatment with clot dissolving drugs, such as tissue plasminogen activators). Of course administration of a compound of the invention should be continued for a period of time after reperfusion has been achieved. Similarly, a compound of the invention should be administered prior to and/or during surgery (e.g., open-heart surgery or surgery to transplant an organ into an animal), and administration of the compound should be continued for a period of time after the surgery. As another example of prophylactic administration, a compound of the invention can be administered to a patient presenting symptoms of a serious condition *(e.g.,* cerebrovascular ischemia or cardiovascular ischemia) while the patient is tested to diagnose the condition. In this way, the patient will be protected during the time it takes to diagnose such conditions, and treatment with the metal-binding compounds of the invention may also prolong the time during which other therapies (e.g., administration of tissue plasminogen activator for cerebrovascular ischemia) can be administered. As yet a further example, a compound of the invention can be administered at the time a patient is to undergo radiation therapy (e.g., radiation for a tumor or prior to a bone marrow transplant).

A compound of the invention can also be used to treat patients who have suffered blunt trauma. In particular, a compound of the present invention may be very beneficial in treating patients suffering from multiple blunt trauma who have a low albumin level, since it has been found that a low albumin level is a predictor of mortality in such patients. More specifically, 34 patients suffering from multiple blunt trauma were studied. These patients were admitted to the intensive care unit of Swedish Hospital, Denver, CO in 1998. Two groups of patients were matched by a trauma surgeon by age, injury severity score (ISS), and type and area of injury without knowledge of the albumin levels of the patients. One group was composed of the patients who died, and the other group was composed of survivors. Following the match, the admission albumin levels were retrieved from the medical records by an independent observer, and the albumin levels of the two groups were compared. For the 17 survivors, the mean albumin level was 3.50 +1.00 g/dl. For the 17 patients who died, the mean albumin level was 2.52 + 0.73 g/dl. The % variance was 28.6 and 28.9, respectively, and the p-value was 0.0026 (95% confidence interval 0.3462 - 0.4771).

The compounds of the invention may be given alone to reduce the damage done by ROS. Alternatively, the compounds of the invention can be given in combination with "free radical scavengers." "Free radical scavengers" include superoxide dismutase, catalase, glutathione peroxidase, ebselen, glutathione, cysteine, N-acetyl cysteine, penicillamine, allopurinol, oxypurinol, ascorbic acid, α-tocopherol, Trolox (water-soluble α-tocopherol), β-carotene, fatty-acid binding protein, fenozan, probucol, cyanidanol-3, dimercaptopropanol, indapamide, emoxipine, dimethyl sulfoxide, and others. *See, e.g.,* Das et al., Methods Enzymol., 233, 601-610 (1994); Stohs, J Basic Cl in. Physiol. Pharmacol., 6,205-228 (1995). The compounds of the invention can, of course, also be given along with standard therapies for a given conditions (e.g., insulin to treat diabetes).

The metal-binding compounds of the invention can also be used to reduce the damage done by ROS in a tissue or organ that has been removed from an animal. To do so, the tissue or organ is contacted with a solution containing an effective amount of a metal-binding compound of the invention. Many suitable solutions are known. See, *e.g.,* Dunphy et al., Am. J. Physiol., 276, H1591-H1598 (1999); Suzer et al., Pharmacol. Res., 37, 97-101 (1998); Hisatomi et al., Transplantation, 52,754-755 (1991*);* U.S. PatentNo. 5,710,172. Effective amounts of the peptide to include in such solutions can be determined empirically, and doing so is within the skill in the art. The harvested tissue or organ may subsequently be used for transplantation into a recipient or for research purposes (e.g., using a perfused liver to screen drugs).

The invention further provides a kit for reducing the damage done by ROS in a tissue or organ that has been removed from an animal. The kit is a packaged combination of one or more containers holding reagents and other items useful for preserving harvested organs. The kit comprises a container holding a metal-binding compound of the invention. Suitable containers include bottles, bags, vials, test tubes, syringes, and other containers known in the art The kit may also contain other items which are known in the art and which may be desirable from a commercial and user standpoint, such as diluents, buffers, empty syringes, tubing, gauze pads, disinfectant solution, etc.

### EXAMPLES

### EXAMPLE 1: Synthesis of Tetrapeptide Asp Ala His Lys [SEQ ID NO:1]

This example describes the synthesis of the tetrapeptide Asp Ala His Lys [SEQ ID N0:1] composed of all L-amino acids using standard solid-phase synthesis techniques. First, 9-fluorenylmethyloxycarbonyl (Fmoc)-protected Asp (v COO- ester; Tolsulfonyl) on Wang resin (0.6 mmole; NovaBiochem) was suspended in a solution ofpiperidine/dimethylformamide (DMF) (40% v/v; 3 ml) for 30 min with occasional agitation. At the end of this period, the solvent was drained, and the resin was washed sequentially with DMF and dichloromethane (DCM; 5 x 3 ml). A ninhydrin test was used to monitor the reaction. The resin was swollen with DMF (~1 ml). The C-protected t-benzyloxycarbonyl (Boc) ester of alanine in DMF was added, followed by a mixture of diisopropylamine (8 equivalent) and 2-(1H-benzotriazole-1-yl)-1,2,3,3-tetramethyluroniumtetrafluoroborate (TBTU-) (4 equivalents). The resin was shaken for about 24 hours, and the reaction was monitored by the ninhydrin test. At the end of this period, DMF was drained, and the resin was washed with DMF and DCM. The solution was drained, and the beads were washed with DCM (3 x 2 ml). The protecting group of the dipeptide-resin was removed, and the beads were suspended in DMF. Amino protected (benzyloxy) derivative of histidine (4 mmole) was added, followed by mixture of diisopropylamine (8 equivalent) and TBTU- (4 equivalent). The resin was shaken for about 24 hours, and the reaction monitored by ninhydrin test. At the end of this period, DMF was drained, and the resin was washed with DMF and DCM. The tripeptide-resin was briefly dried in a gentle stream of nitrogen and suspended in nitrogen-saturated DMF. Protected lysine was added, followed by a mixture of diisopropylamine (8 equivalent) and TBTU- (4 equivalent). The resin was shaken for about 24 hours, and the reaction monitored by the ninhydrin test. At the end of this period, DMF was drained and the resin was washed with DMF and DCM. The Boc protecting group was carefully removed to give the tetrapeptide bound to the resin, with a typical loading of 5 mmole/g. The resin bound tetrapeptide (0.25 gm; 5 mmolar) was treated with trifluoroacetic acid (TFA) and was shaken for 24 hours. At the end of this period, the ninhydrin test gave a blue color, indicating the release of the tetrapeptide from the resin. In some circumstances, addition of 5%(V/V) of DMF to TFA accelerated the rate of release of the peptide from the resin. Removal of TFA at reduced pressure gave the tetrapeptide (all D) as TFA salt and was dried under vacuum at 5°C for 24 hours. The residue was a white powder and was characterized by spectrometric methods.

A number of enantiomers of the tetrapeptide can be prepared in this manner. For example, use of D-amino acids in the peptide synthesis forms the tetrapeptide containing all D-amino acids. Also, combinations of L-amino acids and D-amino acids can be used.

### EXAMPLE 2: Preparation of Cyclohexanediamine

### Derivative of Asp Ala His Lys [SEQ ID NO:1]

Trans-diaminocyclohexane was prepared by resolving cis/trans 1,2-diaminocyclohexane (Aldrich-Sigma) as the tartaric acid salt. The R-trans isomer melts at 75°C and the S-trans isomer melts between 43-45°C (Ph.D. Thesis, P.D. Newman, University College, Cardiff, U.K.,1994). The trans-diaminocyclohexane (10 gm) was then suspended in anhydrous toluene (30mL) and cooled to 5°C in an ice bath, and bromoacetic acid (8 gm) in toluene (25 mL) was added dropwise. At the end of the addition, the reaction temperature was raised to 30°C and kept at that temperature for a further 5 hours. Toluene was evaporated, and the R-trans 1,2-diaminocyclohexane diacetic acid was crystallized from hexane/toluene to give a white solid (yield 70%). The product was characterized by spectroscopic methods.

The resin-bound tetrapeptide prepared in Example 1 (20mg) was suspended in DMF (5 mL) and was treated with the R-trans 1,2-diaminocyclohexanediacetic acid (20 mg) followed by addition of a mixture of diisopropylamine (8 equivalent) and TBTU-(4 equivalent). The resin was shaken for about 24 h on a roller. Then, the resin was washed with DMF followed by DCM (5x3mL) and partially dried. Hydrolysis of the resin linkage was effected by treating the resin-bound reaction product with TFA (5mL); 5 hr). The resin was separated and washed with DCM. The washings were combined with TFA and concentrated under vacuum. The residue (cyclohexanediamine tetrapeptide; formula given in Figure 3D where R₅ is H) was characterized by spectrometric analysis.

### EXAMPLE 3: Preparation of Tetrapeptide Tetracetic Acid

The resin-bound tetrapeptide prepared in Example 1 (20 mg) was suspended in DMF (5 mL) and treated with excess (10-fold) chloroacetic acid. The resin was shaken at room temperature for 48 hours, followed by heating to 60°C for a further hour. DMF was removed by filtration, and the resin was washed with DMF followed by DCM (5x3mL). Partially dried resin was used without further treatment in the next stage. Hydrolysis ofthe resin linkage was effected by treating the resin-bound reaction product with TFA (5mL; 5 hr). The resin was separated and washed with DCM. The washings were combined with TFA and concentrated under vacuum (yield 30%). The product (formula given in Figure 4) was characterized by spectrometric methods.

### EXAMPLE 4: Preparation of Mesoporphyrin IX Tetrapeptide

The resin-bound tetrapeptide prepared in Example 1 (20 mg) was suspended in DMF (5 mL) and treated with mesoporphyrin IX dicarboxylic acid (10 µmole; formula given in Figure 6A), followed by addition of a mixture of diisopropylamine (8 equivalent) and TBTU-(4 equivalent). The resin was shaken for about 24 hours on a roller kept in a dark chamber. The resin was washed with DMF followed by DCM (5x3mL) and partially dried. Hydrolysis of the resin linkage was effected by treating the resin-bound reaction product with TFA (5mL; 5 hr). The resin was separated and washed with DCM/TFA mixture (1:1.5mL). The washings were combined and concentrated under vacuum. The porphyrin tetrapeptide (formula given in Figure 6C) was purified by semi-preparative HPLC (yield 60%). The structure was confirmed by spectrometric methods.

This procedure can be used to synthesize other porphyrin-peptides, such as mesoporphyrin I and related molecules.

### EXAMPLE 5: Preparation of Tetrabispiridylethyl Tetrapeptide

The resin-bound tetrapeptide prepare in Example 1 (20 mg) was suspended in DMF (5 mL) and treated with bromoethylpyridine (20 µmole). This was followed by the addition of pyridine (0.5 mL). The resin was shaken for about 48 hours on a roller. The resin was washed with DMF, followed by DCM (Sx3mL) to remove all of the unreacted monomers, and then dried under vacuum for 30 minutes. Hydrolysis of the resin linkage was effected by treating the resin-bound reaction product with TFA (5mL; 5 hr). The resin was separated and washed with DCM/TFA mixture (1:1.5mL). The washings were combined and concentrated under vacuum. The pyridylethyl tetrapeptide derivative (formula given in Figure 5) was purified by semi-preparative HPLC (yield 50 %). The structure was confirmed by spectrometric methods.

This procedure can be applied to other heterocycles, such as phenanthroline and related molecules.

### EXAMPLE 6: Preparation of Aryl Derivative of Asp Ala His Lys [SEQ ID NO:1]

A derivative having the formula shown in Figure 1B, wherein R₁ is phenyl, was prepared. Diethylacetamidomalonate (10 gm) in anhydrous ethanol (100 mL) was added to a slurry of sodium ethoxide in ethanol (5 gm; 50 mL) and heated to reflux for 30 min. The product was cooled (10°C) and reacted with ethyl α-bromophenyl acetate (5 gm). The reaction was allowed to proceed to completion (24 h), and excess sodium ethoxide was neutralized with dilute acid. The triester was extracted into ethylacetate and, upon removal of solvent, gave a viscous liquid. The crude product was hydrolyzed with hydrochloric acid (100 mL) and decarboxylated to give phenyl substituted aspartic acid (10 gm). The N-benzoyloxy t-butyl derivative was prepared using a standard reaction sequence. To the resin-bound tripeptide (Lys His Ala) prepared as described in Example 1 (20 mg) in DMF was added the N-benzoyloxy-t-butyl aspartic acid derivative, followed by a mixture of diisopropylamine (8 equivalent) and TBTU- (4 equivalent). The resin was shaken for about 24 h, and the reaction monitored by the ninhydrin test. At the end of this period, DMF was drained, and the resin was washed with DMF and DCM. The solution was drained, and the beads washed with DCM (3 x 2 ml). The tetrapeptide derivative was isolated by careful hydrolysis. Stereoisomers of the tetrapeptide were separated by preparative-scale HPLC.

### EXAMPLE 7: Inhibition Of The Generation Of ROS

### By The Tetrapeptide Asp Ala His Lys [SEQ ID NO:1]

A tetrapeptide having the sequence L-Asp L-Ala L-His L-Lys [SEQ ID NO:1] (the L-tetrapeptide) was obtained from one or more companies that provide custom synthesis of peptides, including Ansynth Services, QCB, Genosys and Bowman Research. The peptide was prepared by standard solid phase synthesis methods (see also Example 1).

The ability of the L-tetrapeptide to inhibit the generation of ROS was tested as described in Gutteridge and Wilkins, Biochim. Biophys. Acta, 759, 38-41 (1983) and Cheeseman et al., Biochem. J., 252, 649-653 (1988). Briefly, Cu(II] and H₂O₂ were mixed causing the generation of hydroxyl radicals in a Fenton-type reaction. The hydroxyl radicals attack the sugar 2-deoxy-D-ribose (the sugar residue of DNA) to produce fragments. Heating the fragments at low pH produces malonaldehyde that, upon the addition of 2-thiobarbituric acid, yields a pink chromogen which can be measured spectrophotometrically at 532 nm. Thus, the absorbance at 532 nm is a measure of the damage to 2-deoxy-D-ribose.

The assay was performed with and without the L-tetrapeptide. The results are summarized in Table 1. As can be seen from Table 1, when the L-tetrapeptide was present at Cu(II):tetrapeptide ratios of 1:1.2 and 1:2, the degradation of 2-deoxy-D-ribose was inhibited by 38% and 73%, respectively. Clearly, the L-tetrapeptide inhibited the degradation of 2-deoxy-D-ribose by hydroxyl radicals.

**TABLE 1**

| | CuCl₂ (mM) | H₂O₂ (mM) | Tetra-peptide (mM) | OD at 532 nm | Percent Inhibition |
|---|---|---|---|---|---|
| Control | 0.1 | 2.0 | 0.0 | 0.124 | |
| Tetrapeptide | 0.1 | 2.0 | 0.12 | 0.077 | 38 |
| Control | 0.1 | 2.0 | 0.0 | 0.175 | |
| Tetrapeptide | 0.1 | 2.0 | 0.2 | 0.048 | 73 |

A similar assay was also performed using a tetrapeptide having the sequence Asp Ala His Lys composed of all D-amino acids (D-tetrapeptide). The D-tetrapeptide was obtained from one or more companies that provide custom synthesis of peptides, including Ansynth Services and QCB. The peptide was prepared by standard solid phase synthesis methods (see Example 1)

The ability of the D-tetrapeptide to inhibit the generation of ROS was tested as described by Zhao and Jung, Free Radic Res, 23(3), 229-43 (1995). Briefly, Cu(II) and ascorbic acid were mixed causing the generation of hydroxyl radicals in a Fenton-type reaction. The advantage of using ascorbic acid instead of hydrogen peroxide is that ascorbic acid does not interfere with other assays (i.e. LDH assay) which is not the case with peroxide. The hydroxyl radicals attack the sugar 2-deoxy-D-ribose to produce fragments., Heating the fragments at low pH produces malonaldehyde that, upon the addition of 2-thiobarbituric acid, yields a pink chromogen which can be measured spectrophotometrically at 532 nm. Thus, the absorbance at 532 nm is a measure of the damage to 2-deoxy-D-ribose.

Establishing optimal Cu(II) and ascorbic acid concentrations was the first step in developing this protocol. First, a constant Cu(II) concentration of 10µM was used based on this level being the physiological concentration found in the body (bound and unbound Cu(II)). The ascorbic acid concentrations were varied in order to establish a linear range. The ascorbic acid concentration chosen was 500µM since it gave the most absorbance at 532 nm and still fell in the linear range. Interestingly, at ascorbic acid concentrations greater than 500µM, there was a steady decrease in hydroxyl radicals presumably due to ascorbic acid's dual effect as a hydroxyl radical generator at low concentrations and an antioxidant at high concentrations.

Using the aforementioned concentrations for Cu(II) and ascorbic acid, a titration curve was established for the D-tetrapeptide. Briefly, the D-tetrapeptide was pre-incubated with Cu(II) for 15 minutes at room temperature prior to adding ascorbic acid. This was done to permit the D-tetrapeptide to bind with the Cu(II) and therefore inhibit ROS generation. As can be seen from the table, when the Cu(II):D-tetrapeptide ratio was between 4:1 to 4:7, there was little to no inhibition of hydroxyl radical generation. When the ratio was 1:2 or higher, there was total inhibition of hydroxyl radical production.

**TABLE 2**

| Cu(ll):D-Tetrapeptide | Cu(ll) (µM) | Ascorbic Acid (µM) | D-Tetrapeptide (µm) | A532 | % Inhibition |
|---|---|---|---|---|---|
| 1:0 | 10 | 500 | 0 | 0.767 | |
| 4:1 | 10 | 500 | 2.5 | 0.751 | |
| 2:1 | 10 | 500 | 5 | 0.743 | |
| 1:1 | 10 | 500 | 10 | 0.751 | |
| 4:5 | 10 | 500 | 12.5 | 0.789 | |
| 2:3 | 10 | 500 | 15 | 0.774 | |
| 4:7 | 10 | 500 | 17.5 | 0.737 | |
| 1:2 | 10 | 500 | 20 | 0.029 | 96.2 |
| 1:4 | 10 | 500 | 40 | 0.016 | 97.9 |

### EXAMPLE 8: Testing of Asp Ala His Lys D-Tetrapeptide

### In A Langendorff Reperfusion Model

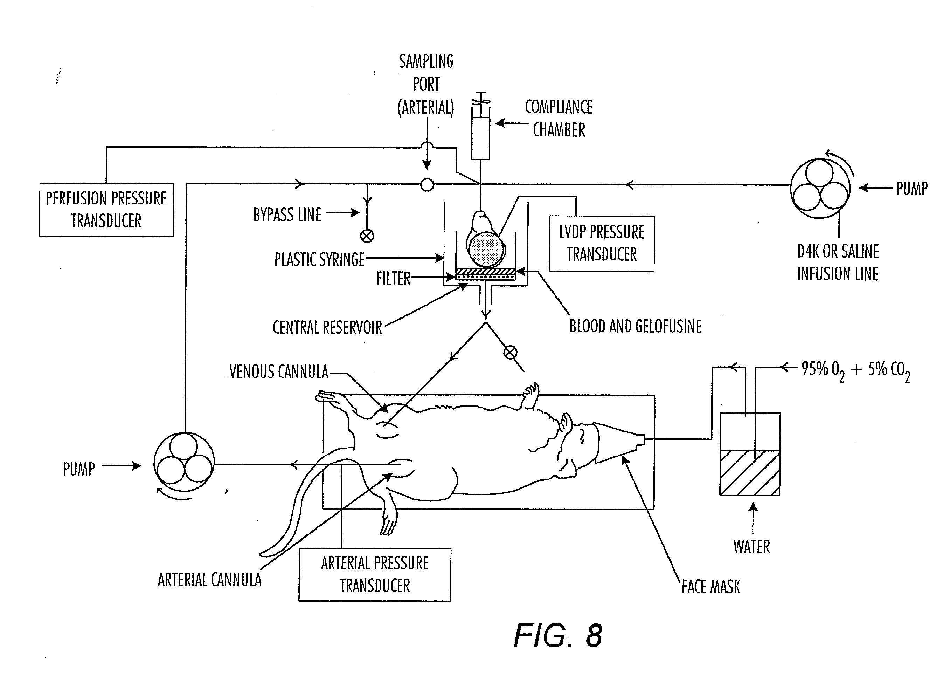

Blood-perfused hearts were prepared essentially as in previous studies (Galiñanes et a 1., Circulation, 88:673-683 (1993); Kolocassides et al., Am. J. Physiol., 269:H1415-H1420 (1995); Hearse et al., J. Mol. Cell. Cardiol., 31:1961-1973 (1999)). See Figure 8. The procedures are described briefly below.

Male Wistar rats, obtained from Bantin and Kingman Universal, UK, were used. All animals received humane care in compliance with the *"*Principles of Laboratory Animal Care" formulated by the National Society for Medical Research and the *"*Guide for the Care and Use of Laboratory Animals" prepared by the National Academy of Sciences, and publisehd by the US National Institutes of Health (NIH Publication No. 85-23, revised 1996).

Support rats (300-400g) were anesthetized with sodium pentobarbitone (60 mg/kg, intraperitoneally) and anticoagulated with heparin (1000 IU/kg intravenously). The right femoral vein and left femoral artery were exposed by blunt dissection and cannulated (18G and 22G Abbocath-T catheters respectively) for the return and supply of blood to the perfused heart. An extracorporeal circuit was established, primed with Gelofusine® plasma substitute (B. Braun Medical Ltd., Aylesbury, UK) and was maintained for 15 minutes (min) before connection to the isolated heart. This period was to ensure that the priming solution was adequately mixed with the blood of the support rat and that the entire preparation was stable. Each 500 ml of Gelofusine® contains 20.00 g succinated gelatin (average molecular weight 30,000), 3.65 g. sodium chloride, water for injection to 500 ml (electrolytes mmol/500 ml: cations Na 77, anions Cl 62.5, pH 7.4). Prior to perfusing the donor heart, an additional 7-8 ml of blood from a rat of the same strain was added to the central reservoir. This was to ensure that the support rat had an adequate supply of blood during the experiment when blood was not recirculated but instead collected for a 2 min period. A peristaltic pump (Gilson Minipuls 3) was located on the arterial outflow of the support rat and flow through the extracorporeal circuit was increased gradually over 10 min to a value of 2.5 ml/min. This gradual increase prevented the drop in arterial pressure that would have occurred if a flow rate of 2.5 ml/min had been established immediately. The blood was pumped through a cannula (to which the aorta of the perfused heart would subsequently be attached) and returned, by gravity, via a reservoir and filter to the venous inflow line of the support animal. An air-filled syringe above the perfusion cannula acted as a compliance chamber, which served to dampen oscillations in perfusion pressure which occurred as a consequence of the contraction of the isolated heart and the peristaltic action of the pump. The support animal was allowed to breathe a mixture of 95% O₂ + 5% CO₂ through a 35% Venturi face mask. The flow rate was adjusted to maintain blood pO₂ and pCO₂ within the physiological range. Body temperature was stabilized at 37.0 (±0.5)°C by means of a thermostatically-controlled heating pad and was monitored by a rectal thermometer. Blood pressure was monitored by means of a pressure transducer attached to the arterial line. All pressure transducers were connected to a MacLab (ADInstruments, Australia), which was run continuously through the experiment. Blood gas (pO₂, pCO₂, pH), hematocrit, glucose and electrolyte levels (Na⁺, K⁺, Ca²⁺) of the support rat were monitored before the donor heart was attached to the extracorporeal circuit and at the end of each experiment. During the course of the experiment, minimum amounts of donor blood (from another at of the same strain) were transfused as required so as to maintain the volume and stability of the preparation. Additional heparin and pentobarbitone were administered into the central reservoir as required.

To isolate hearts, each rat (270-350 g) was anesthetized with diethyl ether and anticoagulated with heparin (1000 IU/kg intravenously). The heart was then immediately excised and immersed in cold (4°C) Gelofusine®. The aorta was rapidly cannulated and perfused in the Langendorff mode, (Langendorff, Pflugers Archives fur die Gestamte Physiologie des Menschen and der Tiere, 61:291-332(1895)) with arterial blood from the support animal, at a constant flow rate of 2.5 ml/min. After removal of the left atrial appendage, a fluid-filled balloon catheter (for the measurements of left ventricular systolic and diastolic pressures, and, by difference, left ventricular developed pressure), attached to a pressure transducer, was introduced into the left ventricle via the mitral valve. The balloon was inflated with water until a left ventricular end diastolic pressure (LVEDP) of between 4-8 mmHg was obtained. Heart rate was calculated from the pressure trace and expressed as beats per minute (bpm). Perfusion pressure was measured via a sidearm of the aortic cannula. All pressure transducers were connected to a MacLab, which was run continuously through the experiment.

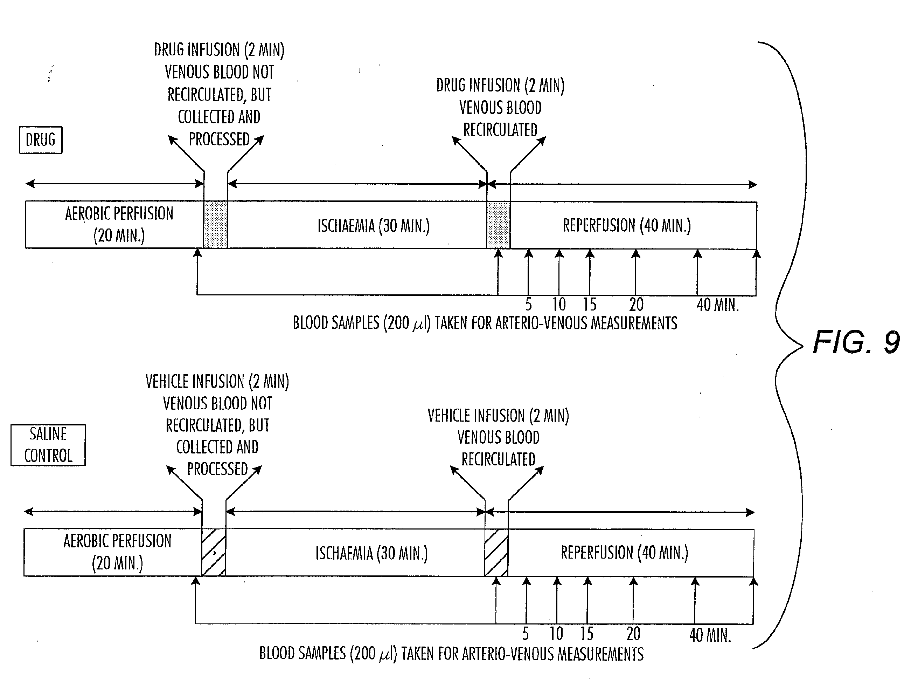

Excised hearts were randomly assigned to two treatment groups (see Figure 9; 6 hearts/group) and aerobically perfused for 20 min prior to (i) saline control with 2 min saline infusion immediately prior to a 3 0 min period of ischemia plus 2 min saline infusion at the onset of reperfusion and (ii) drug with 2 min drug infusion immediately prior to a 30 min period of ischemia (the drug was therefore trapped in the vasculature for the duration of the ensuing ischemic period), plus a 2 min drug infusion at the onset of reperfusion. Hearts were then subjected to 30 min of global, zero-flow ischemia, during which time they were immersed in saline at 37.0°C. Ischemia was initiated by clamping the line leading from the pump to the aortic cannula, thus diverting the flow away from the isolated heart back to the support animal, via the bypass line. Hearts were then reperfused for 40 min, during which time contractile function was continuously measured.

The drug, whose identity was unknown to the researchers performing the experiments, was the tetrapeptide D-Asp D-Ala D-His D-Lys. The tetrapeptide was supplied to the researchers by Bowman Research, UK, dissolved in saline at a concentration of 16.7 mg/mL. It was infused as supplied without any dilution or modification. Physiological saline was supplied by Baxter, UK and used in controls. Fresh solutions of saline and the drug were used daily.

Drug or vehicle was infused into a sidearm of the aortic cannula by means of a peristaltic pump (Gilson Minipuls 3), set at a constant flow of 0.25 ml/min. Since blood flow through the aortic cannula was 2.5 ml/min, and drug infusion was 0.25 ml/min, the final concentration of drug delivered to the heart was 1/11th of that supplied by Bowman Research. During the 2 min period of pre-ischemic vehicle or drug infusion and at the time points indicated in Figure 9 arterial and venous blood samples were collected, centrifuged and frozen for analysis. The infusion was then repeated for the first 2 min of the reperfusion period, during which time the blood was not collected, but recirculated.

Predefined exclusion criteria stated that: (i) support animals would be excluded from the study if they did not attain a stable systolic blood pressure ≥ 80 mm Hg before cannulation of the donor heart, (ii) donor hearts would be excluded from the study if, at the 20 min baseline pre-intervention reading, LVDP ≤ 100 mm Hg or (iii) blood chemistry values were outside the normal range.

Results are expressed as mean ± SEM.. All recovery values are expressed as a percent of the pre-intervention baseline value (measured 20 min after the onset of the experiment) for each individual heart. The two-tailed unpaired Student's *t* test was used for the comparison of two means between groups. A difference was considered statistically significant when p < 0.05.

For reasons of quality control and to allow application of predefined exclusion criteria, the stability and reproducibility of the system were monitored by measuring the blood chemistry (pH, pO₂, pCO₂, haematocrit, Na⁺, K⁺ and Ca²⁺, glucose) and baseline contractile function of each support animal (immediately before perfusing a donor heart and at the end of each experiment) and each perfused heart. Table 3 reveals that there were only minor changes in each index measured, confirming that similar perfusion conditions applied in both study groups and that all values were within the acceptable physiological range. The systolic pressure and heart rate of the support rats are shown in Table 4. As can be seen, there were no significant differences between the two study groups at the 15 min baseline reading.

Table 5 shows that there were no significant differences between groups at the end of the 20 min aerobic perfusion period *(i.e.* just prior to the infusion of drug or vehicle) in LVDP, heart rate and perfusion pressure. Thus, for LVDP, the primary endpoint in the study, the mean values were 177.3 ± 10.6 mmHg and 177.2 ± 5.6 mmHg for the groups that were to become saline control and drug treated.

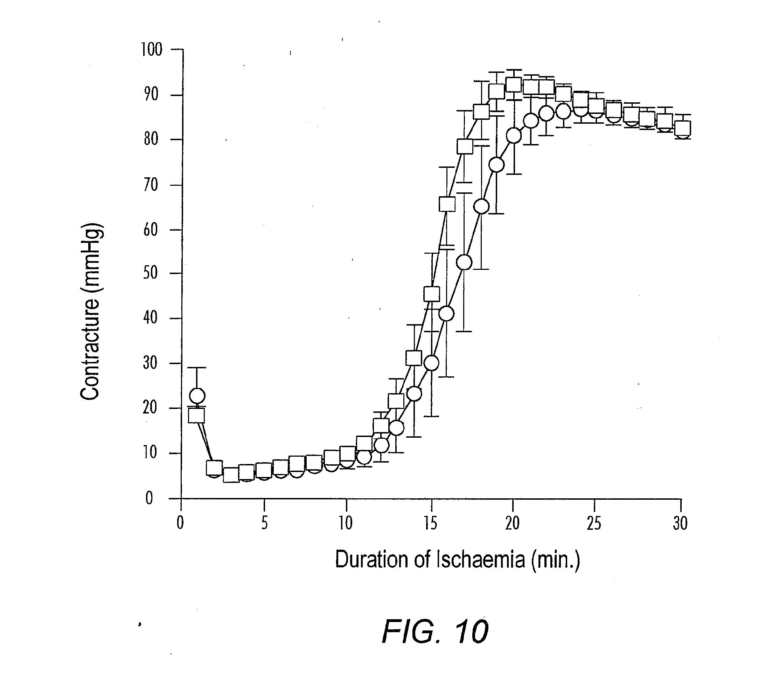

As expected, myocardial ischemia caused cessation ofmyocardial contraction, with cardiac arrest initially in the diastolic state. However, as ischemic injury developed with time an increase in diastolic state occurred as the heart went into ischemic contracture. The temporal profiles for the development of ischemic contracture in each of the study groups is shown in Figure 10. As can be seen from Table 6, there were no differences in any of the measured indices, although there is some evidence of a trend to delay time-to-50% contracture in the drug treated group, which is suggestive with protection.

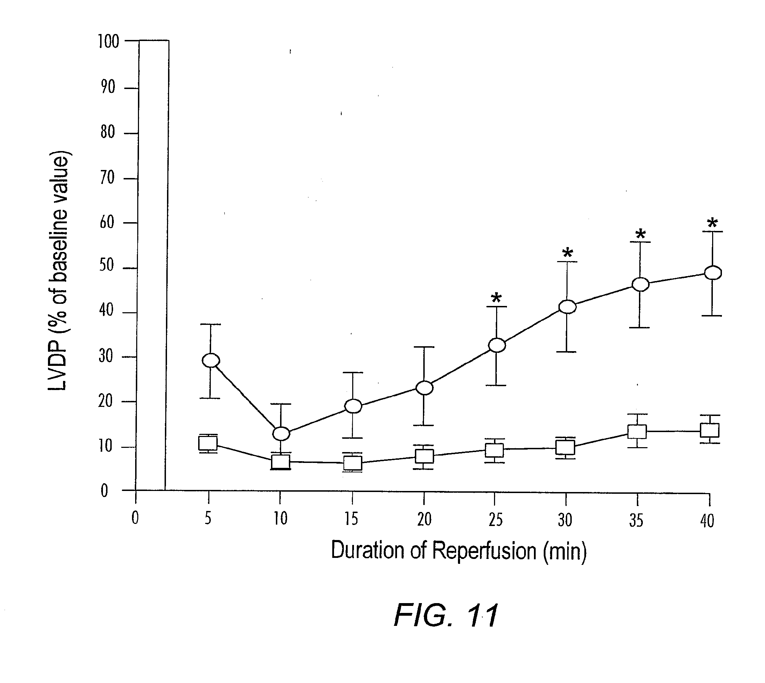

Figure 11 shows the profiles for the mean recovery of LVDP (expressed as a percent of baseline pre-intervention values) in both study groups. It is evident that hearts in the saline control group recovered slowly and poorly, such that by the end of the 40 min reperfusion period, LVDP was only 15.3 ± 3.2% of the pre-intervention control. By contrast, hearts in the drug group recovered more rapidly and to a greater extent (50.5 ± 9.3%).

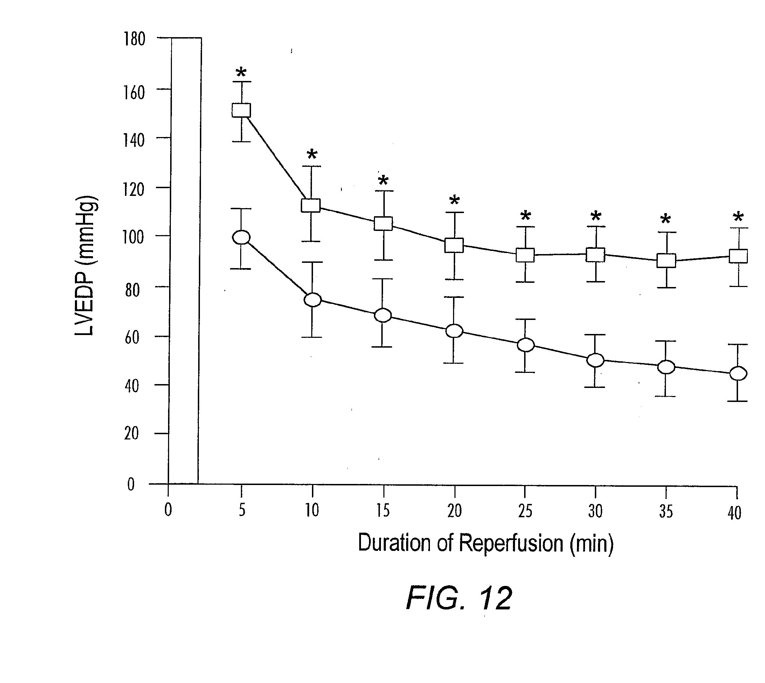

Figure 12 shows the absolute values for the left ventricular end diastolic pressure in both study groups during the 40 min period of reperfusion. In both groups, the high levels of LVEDP resulting from the contracture which developed during ischaemia fell with time towards the pre-intervention control value. However, the drug group normalized their LVEDP more quickly and more completely than that seen in the saline control group, the difference being significant at every time point studied. This would be consistent with the enhanced recovery seen during reperfusion in the drug group.

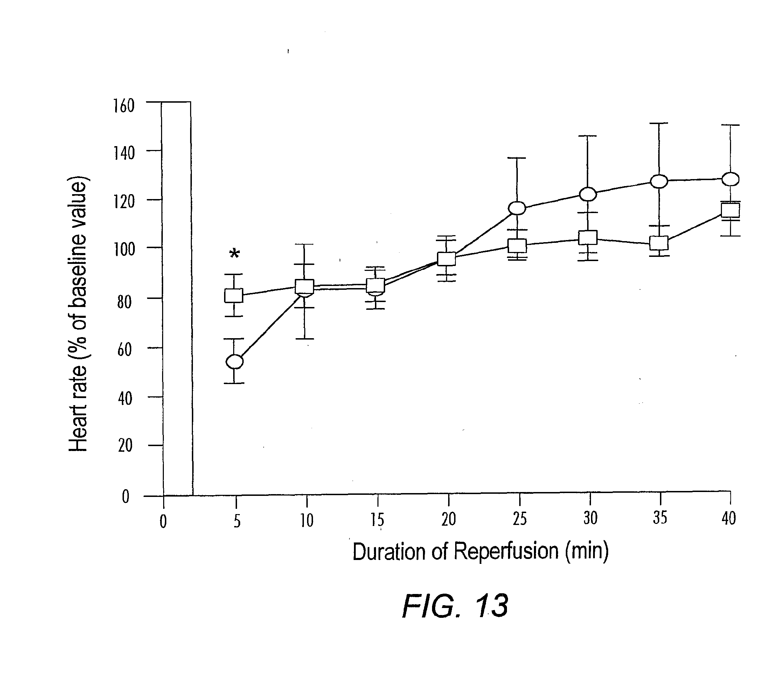

A comparison of the heart rates obtained in the saline control group and drug, as shown in Figure 13 reveals that these two groups were essentially identical (115.0 ± 3.8 versus 127.2 ± 22.8%).

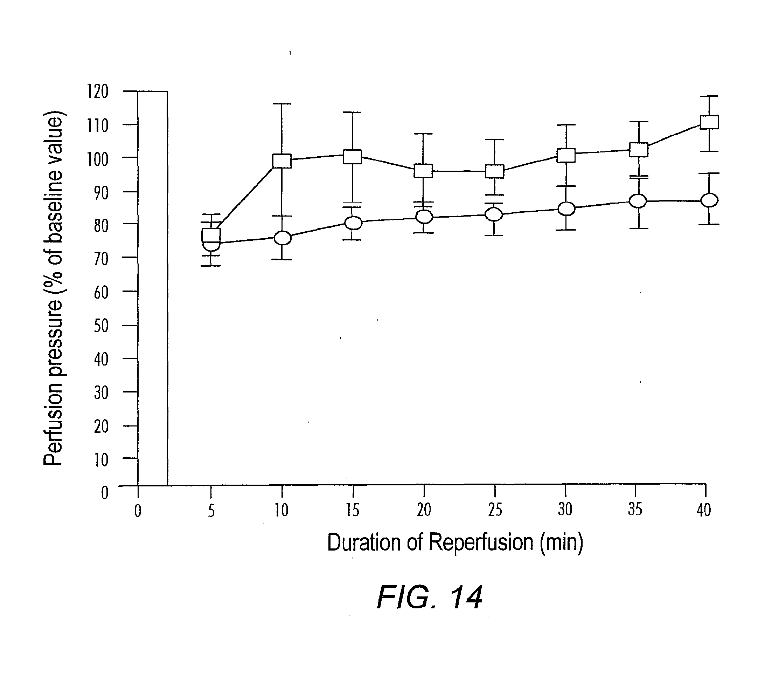

As shown in Figure 14, the perfusion pressure during the 40 min reperfusion period was essentially constant in both groups and the values did not differ significantly between each other at the end of the 40 min reperfusion period. In saline controls, the mean value at the end of reperfusion was 109.9 ± 8.2% of its pre-intervention control, while that of the drug group was 87.4 ± 8.0% of its pre-intervention value. Thus, although the values for drug tended to be lower throughout the reperfusion period (which is consistent with the observed cardioprotection), these changes did not reach statistical significance.

The results of this pilot study indicate that, in the isolated blood-perfused rat heart, Asp Ala His Lys appears to have significant and substantial protective properties as assessed by an approximately three and a half (3.5) fold (15.3 ± 3.2 % to 50.5 ± 9.3%) enhancement of post-ischemic functional recovery. The magnitude of protection is equal to some of the most powerful interventions studied.

**TABLE 3**

| Composition of the blood perfusing the isolated donor hearts | | | | |
|---|---|---|---|---|
| Index | Value prior to attaching donor heart | | End of experiment | |
| | Saline Control | Drug* | Saline Control | Drug* |
| pH | 7.29±0.01 | 7.26±0.01 | 7.33±0.02 | 7.31±0.02 |
| pCO₂ (mmHg) | 60.0±3.2 | 67.7±2.2 | 57.6±4.9 | 65.6±4.7 |
| PO₂ (mmHg) | 230.6±14.8 | 281.7±23.2 | 241.0±37.2 | 252.3±33.9 |
| Haematocrit (%) | 27.5±0.9 | 27.3±1.8 | 26.8±1.7 | 27.0±1.5 |
| Na⁺ (mmol/L) | 146.7±0.3 | 146.2±0.6 | 145.7±0.2 | 146.2±0.5 |
| K⁺ (mmol/L) | 3.3±0.1 | 3.2±0.1 | 4.2±0.2 | 4.2±0.1 |
| Ca²⁺ (mmol/L) | 1.2±0.1 | 1.2±0.1 | 1.4±0.1 | 1.4±0.1 |
| Glucose (mmol/L) | 8.9±0.6 | 10.2±0.3 | 10.2±0.5 | 10.2±0.7 |
| % oxygen saturation | 99.6±0.1 | 99.8±0.1 | 98.7±1.2 | 99.7±0.1 |

| | | | | |
|---|---|---|---|---|
| There were 4-6 support animals per group. All values are expressed as mean ± SEM. * Drug was D-Asp D-Ala D-His D-Lys. | | | | |

**TABLE 4**

| Baseline systolic pressure and heart rate in the support blood-perfused rat | | |
|---|---|---|
| | Control values (t=15 min aerobic perfusion) | |
| Subsequent Treatment | Systolic Pressure (mmHg) | Heart Rate (bpm) |
| Group | | |
| Saline Control | 96.4±2.6 | 296.6±9.8 |
| Drug* | 98.1±3.8 | 297.6±8.7 |

| | | |
|---|---|---|
| All values are expressed as mean ± SEM. There were 6 support animals per group. * Drug was D-Asp D-Ala D-His D-Lys. | | |

**TABLE 5**

| Baseline left ventricular developed pressure (LVDP), heart rate and perfusion pressure in isolated blood-perfused rat hearts before various interventions | | | |
|---|---|---|---|
| | Control values (t=20 min aerobic perfusion) | | |
| Subsequent Treatment | LVDP (mmHg) | Heart Rate | Perfusion Pressure |
| Group | | (bpm) | (mmHg) |
| Saline Control | 177.3±10.6 | 236.6±17.9 | 94.6±7.5 |
| Drug* | 177.2±5.6 | 257.3±29.7 | 99.6±7.3 |

| | | | |
|---|---|---|---|
| All values are expressed as mean ± SEM. There were 6 support animals per group. * Drug was D-Asp D-Ala D-His D-Lys. | | | |

**TABLE 6**

| Ischemic contracture during 30 min of global, zero flow ischaemia | | | | |
|---|---|---|---|---|
| | Contracture | | | |
| Group | Initiation | Time-to-50% | Peak | Time-to-peak |
| | (min) | (min) | (mmHg) | (min) |
| Saline Control | 9.2±1.6 | 15.1±0.5 | 93.7±3.0 | 19.8±0.6 |
| Drug* | 11.9±1.7 | 16.5±0.9 | 89.5±3.0 | 21.7±1.2 |

| | | | | |
|---|---|---|---|---|
| All values are expressed as the mean ± SEM. There were 6 hearts per group. * Drug was D-Asp D-Ala D-His D-Lys. | | | | |

### EXAMPLE 9: Testing of Asp Ala His Lys In A Brain Ischemia Model

Focal ischemic infarcts were made in mature male Wistar rats (270-300 g, Charles River Laboratories) as described previously (Koizumi et al, Jpn. J Stroke, 8:1-8 (1986); Chen et al., J. Cereb.lood Flow Metab., 12(4):621-628 (1992)). Animals were allowed free access to food and water before surgery. They were anesthetized with 3.5% halothane, and anesthesia was maintained with 1.0% - 2.0% halothane in 70% N₂/30% O₂ using a face mask. Rectal temperature was maintained at 37°C during surgery using a feedback-regulated water heating system (YSI 73A rectal probe, Fisher, connected to a K-20/64N aquatic blanket, Hamilton Industries, Cincinnati, OH). Previous studies have shown that rectal and brain temperatures are identical during and after ischemia in this model (Chen et al., J Cereb.lood Flow Metab.,12(4):621-628 (1992)). The right femoral artery was cannulated with medical grade silicone tubing (Technical Products, Inc., Decatur, GA) for measurement of blood gases and blood pressure, and the femoral vein was cannulated for infusions. Cannulae were drawn through a subcutaneous tunnel and exited through the dorsal neck. Arterial blood gases were measured in all animals before and after ischemia.

For ischemia surgery, the right common carotid artery was exposed at its bifurcation. A 4-0 nylon suture, with its tip rounded by heating over a flame, was then advanced 18.5-19.5 mm (depending on the animal's weight) from the external into the internal carotid artery and then through the intracranial carotid artery until the tip occluded the origin of the middle cerebral artery (MCA). Animals were then allowed to awaken from anesthesia. At 2 hours after MCA occlusion, they were re-anaesthetized, and intra-arterial sutures were withdrawn into the external carotid artery.

Beginning one minute prior to occlusion, animals received an intravenous infusion of vehicle alone (control) or drug (D-Asp D-Ala D-His D-Lys) in vehicle over one minute. The identity of the drug was unknown to the researchers performing the experiments. It was supplied to the researchers as a concentrated stock (16.67 mg/ml) in phosphate buffered saline, pH 7.4, and was stored it at -80°C. The drug was determined to be biologically active prior to use by determining its ability to reduce free radical formation *in vitro* as described in Example 7. The stock solution was thawed just prior to use, and a sufficient quantity was administered to give a dose of 20 mg/kg. At the end of the intravenous administration of the drug or vehicle, the nylon suture was immediately advanced to occlude the MCA. Following the 2 hours of occlusion of the MCA, the animals received a repeat intravenous infusion of drug or vehicle over one minute. At the end of the second infusion, the nylon suture was immediately pulled back from occluding the MCA to allow for reperfusion. Also, after the second infusion, the animals were re-anesthetized, and the cannulae were removed. The animals were returned to their home cages, where they were allowed free access to food and water.

Animals were weighed before ischemia and before sacrifice. A neurological examination, as described in Zea Longa et al., Stroke, 20:84-91 (1989), was administered at 1 hour and at 24 hours after reperfusion. Scoring was as follows: 0, normal; 1, failure to extend contralateral (left) forepaw fully (amild focal neurologic deficit); 2, circling to the left (a moderate focal nuerologic deficit); 3, falling to the left (a several focal neurologic deficit); and 4, no spontaneous gait and depressed level of consciousness.

Twenty-four hours after MCA occlusion, animals were anesthetized with ketamine (44 mg/kg) and xylazine (13 mg/kg), both given intramuscularly, and perfused transcardially with heparinized saline, followed by 10% buffered formalin. The brains were removed and cut into 2-mm coronal slices using a rat brain matrix (Activational System, Inc., Warren, MI; a total of 7 slices). The slices were then embedded in paraffin, and 6-mm sections were cut from the anterior surface of each slice and stained with hematoxylin and eosin (H and E). Infarct volume was determined using a computer-interfaced image analysis system (Global Lab Image system, Data Translation, Marlboro, MA), using the "indirect" method (Swanson et al... J Cerebral Blood Flow Metabol.,10:290-293 (1990)): the area of intact regions of the ipsilateral (right) hemisphere and area of the intact contralateral (left) hemisphere were determined for each slice, the former was substracted from the latter to calculate infract area per slice. Infarct areas were then summed and multiplied by slice thickness to yield infarct volume per brain (in mm³).

The results are presented in Tables 7-10 below. Some of the data are expressed as mean ± S.E.M. Continuous data were analyzed by repeated measures ANOVA and paired or unpaired two-tailed t-tests with Bonferroni correction where appropriate. Non-continuous behavior date were analyzed by the Mann-Shitney U-test.

**TABLE 7**

| Infarct Volume | | | |
|---|---|---|---|
| Treated* | | Control | |
| Animal | Infarct Volume (mm³) | Animal | Infarct Volume (mm³) |
| #1 | 11.5 | #2 | 44.5 |
| #3 | 14.7 | #4 | 32.3 |
| #5 | 43.3 | #6 | 39.4 |
| #7 | 10.9 | #8 | 22.2 |
| Mean | 20.1 | Mean | 34.6 |
| S. E. M. | 7.7 | S. E. M. | 4.8 |

| | | | |
|---|---|---|---|
| * Treated with D-Asp D-Ala D-His D-Lys | | | |

**TABLE 8**

| Neurological Scale | | | | | |
|---|---|---|---|---|---|
| **Treated*** | | | **Control** | | |
| Animal | Day 0^{a} | Day 1^{b} | Animal | Day O^{a} | Day 1^{b} |
| #1 | 2 | 1 | #2 | 2 | 2 |
| #3 2 | | 1 | #4 | 2 | 2 |
| #5 | 2 | 2 | #6 | 2 | 2 |
| #7 | 2 | 1 | #8 | 2 | 2 |

| | | | | | |
|---|---|---|---|---|---|
| * Treated with D-Asp D-Ala D-His D-Lys a Day of ischemia surgery b One day after ischemia surgery | | | | | |

**TABLE 9**

| Body Weight | | | | | |
|---|---|---|---|---|---|
| Treated* | | | Control | | |
| Animal | Day 0^{a} | Day 1^{b} | Animal | Day O^{a} | Day 1^{b} |
| #1 | 300 | 272 | #2 | 300 | 251 |
| #3 | 300 | 266 | #4 | 300 | 256 |
| #5 | 295 | 256 | #6 | 278 | 230 |
| #7 | 295 | 255 | #8 | 300 | 250 |

| | | | | | |
|---|---|---|---|---|---|
| * Treated with D-Asp D-Ala D-His D-Lys a Day of ischemia surgery b One day after ischemia surgery | | | | | |

**TABLE 10**

| Blood Gases And Blood Pressure | | | | | | | | | |
|---|---|---|---|---|---|---|---|---|---|
| **10 minutes before MCA occlusion** | | | | | **10 minutes after MCA occlusion** | | | | |
| Animal | pH | pCO₂ | pO₂ | BP^{c} | Animal | pH | pCO₂ | pO₂ | BP^{c} |
| #1 | 7.437 | 38.5 | 117.1 | 96 | #1 | 7.422 | 40.2 | 130.1 | 106 |
| #3 | 7.430 | 38.3 | 110.5 | 89 | #3 | 7.423 | 40.1 | 130.1 | 98 |
| #5 | 7.518 | 38.5 | 147.0 | 98 | #5 | 7.471 | 36.5 | 105.3 | 92 |
| #7 | 7.423 | 33.4 | 97.4 | 86 | #7 | 7.433 | 34.0 | 139.9 | 89 |
| #2 | 7.401 | 35.5 | 120.1 | 90 | #2 | 7.423 | 40.1 | 130.1 | 103 |
| #4 | 7.440 | 38.5 | 121.1 | 93 | #4 | 7.421 | 39.5 | 129.3 | 101 |
| #6 | 7.425 | 35.9 | 110.0 | 88 | #6 | 7.453 | 36.2 | 105.3 | 92 |
| #8 | 7.417 | 39.5 | 120.7 | 90 | #8 | 7.428 | 36.9 | 111.0 | 93 |

### EXAMPLE 10: Inhibition Of The Generation Of ROS

The ability of the tetrapeptide L-Asp L-Ala L-His L-Lys [SEQ ID N0:1] and other peptides and compounds to inhibit the production of ROS was tested. The other peptides tested were: L-Asp L-Ala L-His L-Lys L-Ser L-Glu L-Val L-Ala L-His L-Arg L-Phe L-Lys [SEQ ID N0:3]; L-Ala L-His L-Lys L-Ser L-Glu L-Val L-Ala L-His L-Arg L-Phe L-Lys [SEQ ID N0:4]; L-His L-Lys L-Ser L-Glu L-Val L-Ala L-His L-Arg L-Phe L-Lys [SEQ ID NO:5]; and Acetylated-L-Asp L-Ala L-His L-Lys L-Ser L-Glu L-Val L-Ala L-His L-Arg L-Phe L-Lys [SEQ ID NO:6]. The peptides were obtained from one or more companies that provide custom synthesis of peptides, including Ansynth Services, QCB, Genosys and Bowman Research. The other compounds tested were histidine (Sigma Chemical Co.), catalase (Sigma Chemical Co.), and superoxide dismutase (Sigma Chemical Co.).

### 1. Inhibition Of Hydroxyl Radical Production

The hydroxyl radical is probably the most reactive oxygen-derived species. The hydroxyl free radical is very energetic, short-lived and toxic.

Some researchers suggest that the toxicity of hydrogen peroxide and superoxide radical may be due to their conversion to the hydroxyl free radical. The superoxide radical can be directly converted to the hydroxyl radical via the Haber-Weiss reaction. Alternatively, it can be converted to hydrogen peroxide which, in turn, is converted into the hydroxyl radical via the Fenton reaction. Both pathways require a transition metal, such as copper (Acworth and Bailey, The Handbook Of Oxidative Metabolism (ESA, Inc. 1997)).

It is also known that copper, in the presence of ascorbate, produces hydroxyl radicals. The following reaction scheme has been suggested:

Ascorbate + 2Cu²⁺ → 2Cu⁺ + dehydroascorbate + 2H⁺ (Eq.1)

Cu⁺ + O₂ → O₂^{.-} + Cu²⁺ (Eq.2)

Cu⁺ + O₂^{.-} + 2H⁺ → Cu²⁺ + H₂O₂ (Eq.3)

Cu⁺ + H₂O₂ → OH⁻ + OH^{.} + Cu²⁺ (Eq.4)

The ability of the compounds listed above to inhibit the generation of hydroxyl radicals was tested as described in Gutteridge and Wilkins, Biochim. Biophys. Acta, 759:38-41 (1983). Briefly, Cu(II) and ascorbic acid were mixed causing the generation of hydroxyl radicals. Then, deoxyribose was added, and the hydroxyl radicals, if present, attacked the deoxyribose to produce fragments. Heating the fragments at low pH produced malonaldehyde that, upon the addition of 2-thiobarbituric acid (TBA), yielded a pink chromogen which was measured spectrophotometrically at 532 nm. Thus, absorbance at 532 nm is a measure of the damage to deoxyribose and, therefore, of hydroxyl radical formation.

To perform the assay, CuCl₂ in buffer (20 mM KH₂PO₄ buffer, pH 7.4) and either one of the test compounds in buffer or buffer alone were added to test tubes (final concentration of CuCl₂ was 1 0µM). The test tubes were incubated for 15 minutes at room temperature. Then, 0.5 mM ascorbic acid in buffer and 1.9 mM 2-deoxy-D-ribose in buffer were added to each test tube, and the test tubes were incubated for 1 hour at 37°C. Finally, 1 ml of 1% (w/v) TBA in 50 mM NaOH and 1 ml of concentrated acetic acid were added to each test tube, and the test tubes were incubated in boiling water for 15 minutes. After the test tubes had cooled for 15 minutes, the absorbance at 532 nm was read.

It was found that the tetrapeptide L-Asp L-Ala L-His L-Lys [SEQ ID N0:1] caused complete inhibition of the formation ofhydroxyl radicals in this assay at tetrapeptide/copper ratios of 2:1 or higher. Tetrapeptide/copper ratios less than 2:1 were ineffective.

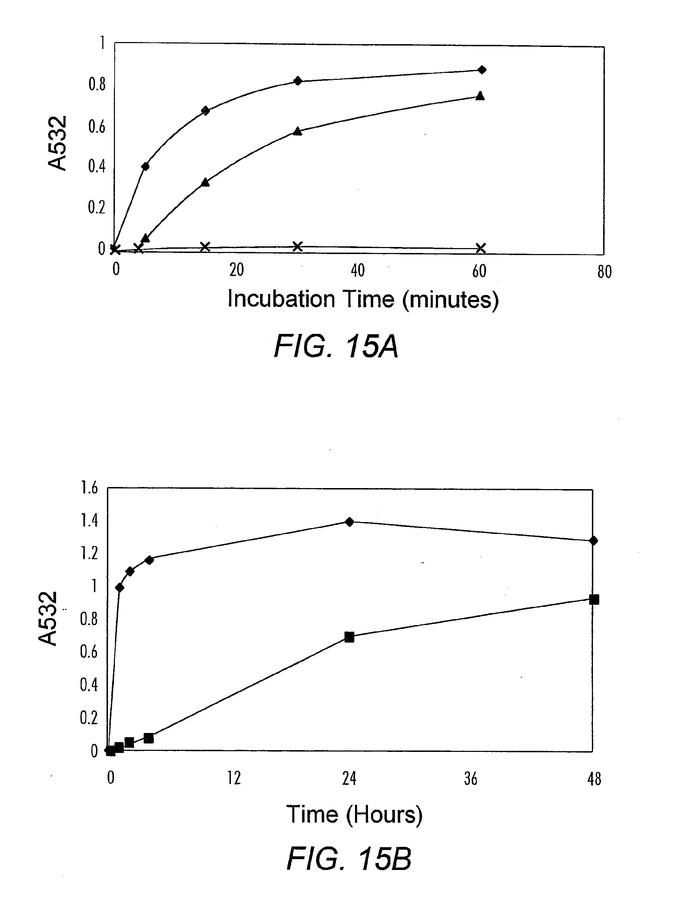

The results of a time course are presented in Figure 15A. As can be seen in Figure 15A, copper and ascorbate (no added peptide) produced TBA-reactive substances quickly and reached a maximum in 30 minutes. The tetrapeptide at a tetrapeptide/copper ratio of 2:1 prevented all formation of TBA-reactive substances. Interestingly, the tetrapeptide at a tetrapeptide/copper ratio of 1:1 slowed the production of TBA-reactive substances. These data suggest that the tetrapeptide at a 1:1 tetrapeptide/copper ratio is able to offer some protection from hydroxyl radicals by binding copper which results in site-directed hydroxyl attack on the tetrapeptide. Once enough of the tetrapeptide is destroyed, then copper is released, which allows it to produce hydroxyl radicals that attack the dexoyribose.

When the tetrapeptide at a tetrapeptide/copper ratio of 2:1 was incubated for longer periods of time, its ability to prevent the formation of TBA-reactive substances slowly eroded. See Figure 15B. As can be seen from Figure 15B, the production of TBA-reactive substances was inhibited by 95% during the first 4 hours of incubation. By 24 hours, the level of inhibition had dropped to 50% and, by 48 hours, the level of inhibition had dropped to 20%. These data suggest that TBA-reactive substances are still being produced even in the presence of the tetrapeptide. They also suggest that the tetrapeptide is being degraded during the time course of the experiment. This degradation is more than likely due to the formation of free radicals in close proximity to the tetrapeptide/copper complex which attack and degrade the tetrapeptide, with release of the copper. Since free radicals, such as the hydroxyl radical, are very reactive, they will attack the first electron rich molecule they come into contact with, which would be the tetrapeptide in this case.

The effect of pH on the inhibition of hydroxyl radical formation by the tetrapeptide was tested at a tetrapeptide/copper ratio of 2:1. At this ratio, the tetrapeptide gave 95% inhibition of the formation of TBA-reactive species at pH 7.0-8.5. These are physiological pH levels and pH levels that would be expected during ischemia (acidosis occurs in ischemic tissues). At pH 6.0, the tetrapeptide was ineffective at preventing the formation of TBA-reactive species, possibly due to the reduced ability of the histidine to bind copper. The nitrogen atom on the imidazole ring of histidine participates in binding copper with a pKa of 6.0. Therefore, at a pH of 6.0, histidine is only able to bind 50% of the copper. The other 50% of the copper would be unbound or loosely bound to the tetrapeptide by the other amino acids and would, therefore, be able to participate in the production of TBA-reactive species.

Histidine and several peptides with histidine in different positions were tested at 1:1 and 2:1 peptide:copper ratios for their ability to inhibit the production of hydroxyl radicals. Also, a peptide having an acetylated aspartic acid (Ac-Asp) as the N-terminal amino acid was also tested. The results are presented in Table 11. In Table 11, the % inhibition is the percent decrease in absorbance compared to buffer alone divided by the absorbance of the buffer alone.

As can be seen from the results in Table 11, the peptides with histidine in the second and third positions gave 95% inhibition at a 2:1 peptide:copper ratio, while these peptides at a 1:1 peptide:copper ratio were ineffective. Interestingly, at a 2:1 peptide:copper ratio, the peptide with histidine in the first position and the peptide with acetylated aspartic acid as the N-terminal amino acid provided some protection (about 47% and about 28% inhibition, respectively), although this protection might be attributable to the histidine in the seventh and ninth positions, respectively, of these peptides. Histidine alone at a 2:1 histidine:copper ratio provided some protection (about 20% inhibition).

Catalase has been shown to prevent hydroxyl radical formation. Gutteridge and Wilkins, Biochim. Biophys. Acta, 759:38-41 (1983); Facchinetti et al., Cell. Molec. Neurobiol.,18(6):667-682 (1998); Samuni et al., Eur. J. Biochem., 137:119-124 (1983). Catalase (0-80 nM) was, therefore, tested in this assay, and it was found to prevent the formation of the pink chromogen (data not shown). This finding suggests that hydrogen peroxide is formed in this assay, since catalase breaks down hydrogen peroxide to water and agrees with Equations 3 and 4 above. Catalase also prevents the formation of the pink chromogen when the L-Asp L-Ala L-His L-Lys [SEQ ID NO: 1] tetrapeptide at a tetrapeptide/copper ratio of 1:1 is present (data not shown). As shown above, at this ratio, the copper is still able to participate in the redox reactions to produce hydroxyl radicals. These experiments show that hydrogen peroxide is an important precursor to the formation of the hydroxyl radical.

**TABLE 11**

| **Compound (Ratio)^{a}** | **Absorbance at 532 nm** | **Absorbance at 532 nm** | **% Inhibition** |
|---|---|---|---|
| Copper only (buffer control) | 0.767* | 0.954 | 0 |
| Histindine/copper (2:1) | | 0.760 | 20.3 |
| His Lys Ser Glu Val Ala His Arg Phe Lys^{b}/copper(1:1) | | 0.716 | 24.9 |
| His Lys Ser Glu Val Ala His Arg Phe Lys^{b}/copper (2:1) | | 0.509 | 46.6 |
| Ala His Lys Ser Glu Val Ala His Arg Phe Lys^{c}/copper(1:1) | | 0.843 | 11.6 |
| Ala His Lys Ser Glu Val Ala His Arg Phe Lys^{c}/copper (2:1) | | 0.047 | 95.1 |
| Asp Ala His Lys Ser Glu Val Ala His Arg Phe Lys^{d}/copper (1:1) | 0.645 | | 13.2 |
| Asp Ala His Lys Ser Glu Val Ala His Arg Phe Lys^{d}/copper (2:1) | 0.040 | | 95.8 |
| Ac-Asp Ala His Lys Ser Glu Val Ala His Arg Phe Lys^{e}/copper (1:1) | 0.633 | | 16.9 |
| Ac-Asp Ala His Lys Ser Glu Val Ala His Arg Phe Lys^{e}/copper (2:1) | 0.692 | | 27.5 |
| Asp Ala His Lys^{f}/copper (1:1) | 0.751* | | 1.3 |
| Asp Ala His Lys^{f}/copper (2:1) | 0.029* | | 96.2 |

| | | | |
|---|---|---|---|
| ^{a} All amino acids are L-amino acids ^{b} SEQ ID NO:5 ^{c} SEQ ID NO:4 ^{d} SEQ ID NO:3 ^{e} SEQ ID NO:6 ^{f} SEQ ID NO:1 * Data taken from Table 2 in Example 7. | | | |

### B. Assay For Superoxide Dismutase (SOD) Activity

The enzyme superoxide dismutase (SOD) is a naturally-occurring enzyme which is responsible for the breakdown in the body of superoxide to hydrogen peroxide (similar to Equation 3). Hydrogen peroxide can then be detoxified by catalase.

SOD was assayed for activity in the assay described in the previous section and was found to have none (data not shown). This result is not surprising since SOD actually converts superoxide radical into hydrogen peroxide. Hydrogen peroxide can then be converted into the hydroxyl radical by reduced copper.

There are reports in the literature that copper complexes have SOD activity. Athar et al., Biochem. Mol. Biol. Int., 39(4):813-821 (1996); Ciuffi et al., Pharmacol Res., 38(4):279-287 (1998); Pogni et al., J. Inorg. Biochem., 73:157-165 (1999); Willingham and Sorenson, Biochem. Biophys. Res. Commun.,150(1):252-258 (1988); Konstantinova et al., Free Rad. Res. Comms.,12-13:215-220 (1991); Goldstein et al J. Am. Chem. Soc.,112]:6489-6492 (1990). This fmding is not surprising since SOD itself has copper in its active site.

The SOD activity of copper complexes of the tetrapeptide L-Asp L-Ala L-His L-Lys [SEQ ID NO:1] was assayed. Superoxide radicals were produced using the xanthine oxidase assay of Beauchamp and Fridovich, Anal. Biochem., 44:276-287 (1971). Xanthine oxidase converts xanthine into uric acid, with oxygen acting as an electron acceptor. This causes superoxide radical to be produced. Superoxide radical is able to reduce nitro blue tetrazolium (NBT). Reduced NBT has a λmax of 560 nm. It is known that copper inhibits xanthine oxidase activity (Konstantinova et al., Free Rad. Res. Comms.,12-13:215-220 (1991)), so all experiments containing copper also contained ethylenediaminetetracetic acid (EDTA), a known copper chelator. The EDTA-copper complex was tested for SOD activity and was shown to have no SOD activity (data not shown).

To perform the assay for SOD activity, 0.1 mM xanthine (Sigma Chemical Co.), 25 µM NBT (Sigma Chemical Co.), 50 mM sodium carbonate, and 1.2 µM EDTA (Sigma Chemical Co.), were mixed in a cuvette (all final concentrations, final pH 10.2). The reaction was started by the addition of various amounts of a tetrapeptide-copper complex (tetrapeptide/copper ratios of 1:1 and 2:1) and 20 nM xanthine oxidase (Sigma Chemical Co.). The tetrapeptide-copper complex was prepared by mixing the tetrapeptide and copper (as CuCl₂) and allowing the mixture to incubate for 15 minutes at room temperature immediately before addition to the cuvette. The samples were read at time 0 and every 60 seconds for five minutes at 560 nm.

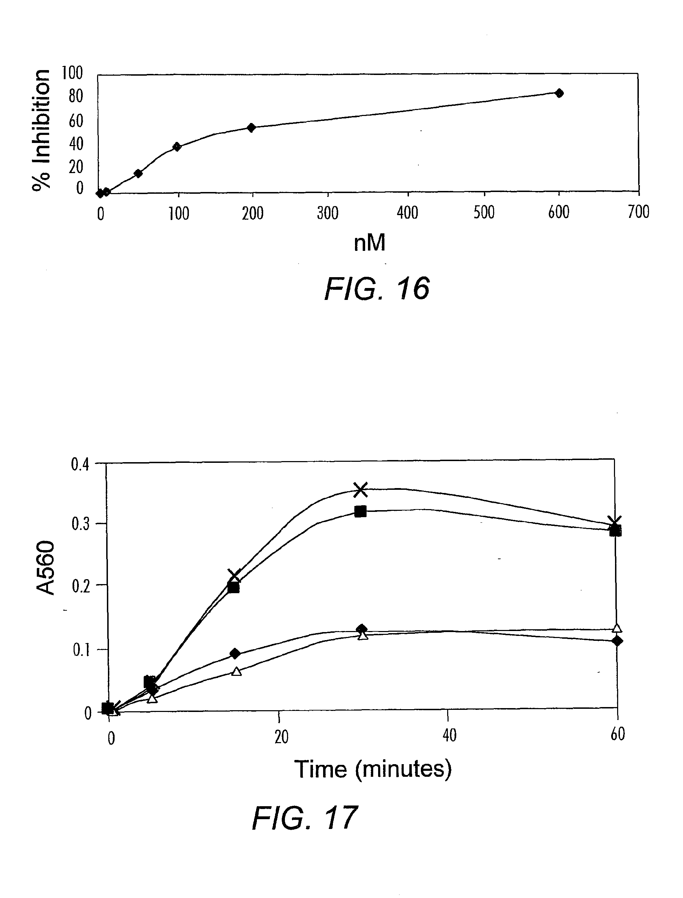

The complex of the tetrapeptide with copper at a ratio of 1:1 was shown to have SOD activity, as evidenced by inhibition of NBT reduction (see Figure 16). However, the complex was about 500 times less effective than SOD itself, based on IC₅₀ values (amount that gives 50% inhibition) in this assay. The complex of the tetrapeptide with copper at a ratio of 2:1 was found to have no SOD activity (data not shown).

To verify that the 1:1 tetrapeptide-copper complex did not interfere with xanthine oxidase activity, uric acid production was measured at 295 nm. Athar et al., Biochem. Mol. Biol. Int., 39(4):813-821 (1996); Ciuffi et al., Pharmacol Res., 38(4):279-287 (1998). This assay is similar to the SOD assay, except that NBT is not present. Instead, uric acid is assayed at 295 nm every 60 seconds for 5 minutes. It was found that the 1:1 tetrapeptide-copper complex only inhibited uric acid production by 11% at a concentration of 600 nM (data not shown). Therefore, the 1:1 tetrapeptide-copper complex has true SOD activity. Since superoxide is converted to hydrogen peroxide by the complex, this could help to explain why it is not effective at preventing hydroxyl radical production.

Superoxide radical production was measured in solutions containing the 1:1 or 2:1 tetrapeptide-copper complexes. The assay combined techniques from the TBA assay and the xanthine oxidase assay. NBT was added to all test tubes in order to quantitate its reduction by superoxide radical. The samples also contained ascorbate and copper and were incubated at 37°C. At 5,15,30 and 60 minutes, the samples were removed from the incubator and read at 560 nm. The results are shown in Figure 17. In the sample containing the 2:1 tetrapeptide-copper complex, NBT reduction increased over time and reached a maximum at 3 0 minutes. The sample containing the 1:1 tetrapeptide-copper complex also showed an increase in NBT reduction, with a decreased maximum reached at 60 minutes. These data suggest that superoxide accumulates in the sample containing the 2:1 tetrapeptide-copper complex, while the 1:1 tetrapeptide-copper complex mimics superoxide dismutase.

The likely sequence of events that occurs in the production of hydroxyl radicals is as follows:

O₂ → O₂^{.-} → H₂O₂ → OH^{.} (Eq.5).

It has already been shown that the 1:1 tetrapeptide-copper complex can convert superoxide radical (O₂ ) into hydrogen peroxide (H₂O₂). This is the SOD activity of the complex. The 2:1 tetrapeptide-copper complex cannot facilitate this conversion since the two molecules of the tetrapeptide fill all six coordination bonds of copper. This explains why the 2:1 tetrapeptide-copper complex is so effective because it inhibits the formation of hydrogen peroxide, which could in turn react with reduced copper to produce hydroxyl radicals via the Fenton reaction. The 1:1 tetrapeptide-copper complex also provides a valuable service by eliminating the superoxide radical. Even though it produces hydrogen peroxide, most compartments of the human body have sufficient quantities of the enzyme catalase that can eliminate hydrogen peroxide. In the brain, however, catalase activity is reported to be minimal. Halliwell et al., Methods in Enzymol.,186:1-85 (1990). Therefore, the brain is a particularly vulnerable organ during periods of ischemia, since copper is released due to the acidosis that accompanies ischemia.

### C. Protection of DNA

DNA strand breaks were measured according to the method of Asaumi et al., Biochem. Mol. Biol. Int., 39(1):77-86 (1996). Briefly, 17 µg/ml of plasmid pBR322 DNA was allowed to pre-incubate for 15 minutes at room temperature with 50 µM CuCl₂ and concentrations of the tetrapeptide of 0-200 µM. Then, 2.5 mM ascorbate was added to each reaction, and the mixture was incubated for 1 hour at 37°C. The total volume of the mixture was 16 µL. Next, 3 µL of loading buffer containing 0.25% (w/v) bromophenol blue, 0.25% (w/v) xylene cyanole FF, and 40% (w/v) sucrose in water was added. The samples were separated by electrophoresis in a 0.8% agarose gel for 90 minutes at 70 Volts. The gel was stained in 1X TBE (Tris-Borate-EDTA buffer) containing 2 µg/ml ethidium bromide for 30 minutes. The gel was then destained in 1X TBE for 5 minutes prior to photographing the gel.

The results showed that the tetrapeptide was very effective at preventing the formation of DNA strand breaks. See Figure 18. Optimal protective tetrapeptide:copper ratios were 2:1 and greater, since superhelical circular DNA was still visible on the gel at these ratios. At a tetrapeptide:copper ratios of 1:1 or less, nicked circular DNA, linear DNA and more damaged DNA (smears) were visible.

## Claims

1. A metal-binding peptide or a physiologically-acceptable salt thereof, wherein the peptide has two binding sites for transition metal ions and the peptide has the formula:
P₁-P₂,
wherein:
P₁ provides one of the binding sites for a transition metal ion, and P₂ provides the other binding site;
P₁ is:
Xaa₁ Xaa₂ His or
Xaa₁ Xaa₂ His Xaa₃;
Xaa₁ is glycine, alanine, valine, leucine, isoleucine, serine, threonine, aspartic acid, asparagine, glutamic acid, glutamine, lysine, hydroxylysine, histidine, arginine, ornithine, phenylalanine, tyrosine, tryptophan, cysteine, methionine, or α-hydroxymethylserine;
Xaa₂ is glycine, alanine, *β*-alanine, valine, leucine, isoleucine, serine, threonine, aspartic acid, asparagine, glutamic acid, glutamine, lysine, hydroxylysine, histidine, arginine, ornithine, phenylalanine, tyrosine, tryptophan, cysteine, methionine, or α-hydroxymethylserine;
Xaa₃ is glycine, alanine, valine, lysine, arginine, ornithine, aspartic acid, glutamic acid, asparagine, glutamine or tryptophan; and
P₂ is an amino acid sequence of no more than 10 amino acids that comprises an amino acid sequence that binds a transition metal ion.

2. The peptide of Claim 1 wherein Xaa₁ is aspartic acid, glutamic acid, arginine or α-hydroxymethylserine.

3. The peptide of Claim 1 wherein Xaa₂ is glycine, alanine, valine, leucine, isoleucine, threonine, serine, asparagine, methionine, histidine or α-hydroxymethylserine.

4. The peptide of Claim 1 wherein Xaa₃ is lysine.

5. The peptide of Claim 1 wherein:
Xaa₁ is aspartic acid, glutamic acid, arginine or α-hydroxymethylserine;
Xaa₂ is glycine, alanine, valine, leucine, isoleucine, threonine, serine, asparagine, methionine, histidine or α-hydroxymethylserine; and
Xaa₃, when present, is lysine.

6. The peptide of Claim 5 wherein:
Xaa₁ is aspartic acid or glutamic acid; and
Xaa₂ is glycine, alanine, valine, threonine, serine or α-hydroxymethylserine.

7. The peptide of Claim 6 wherein Xaa₂ is alanine, threonine or α-hydroxymethylserine.

8. The peptide of Claim 7 wherein Xaa₁ is aspartic acid and Xaa₂ is alanine.

9. The peptide of any one of Claims 1-8 wherein P₂ comprises one of the following sequences:
(Xaa₄)ₘ Xaa₃ His Xaa₂ Xaa₅,
(Xaa₄)ₘ His Xaa₂ Xaa₅,
(Xaa₄)ₘ Xaa₅ Xaa₂ His Xaa₃, or
(Xaa₄)ₘ Xaa₅ Xaa₂ His,
wherein:
Xaa₂ and Xaa₃ are defined in Claim 1,
Xaa₄ is any amino acid,
Xaa₅ is an amino acid having a free side-chain -NH₂, and
m is 0-5.

10. The peptide of Claim 9 wherein Xaa₅ is Orn or Lys.

11. The peptide of any one of Claims 1-8 wherein the transition metal ion is a copper ion.

12. The peptide of any one of Claims 1-8 wherein P₂ comprises one of the following sequences:
Met Xaa₄ Met
Met Xaa₄ Xaa₄ Met
Cys Cys
Cys Xaa₄ Cys
Cys Xaa₄ Xaa₄ Cys
Met Xaa₄ Cys Xaa₄ Xaa₄ Cys
Met Thr Cys Xaa₄ Xaa₄ Cys, or
Gly Met Thr Cys Xaa₄ Xaa₄ Cys,
wherein Xaa₄ is any amino acid.

13. The peptide of any one of claims 1-12 wherein at least one of the amino acids of P₁ other than glycine or *β*-alanine, when present, is a D-amino acid.

14. The peptide of any one of claims 1-13 wherein at least one of the amino acids of P₂ other than glycine or *β*-alanine, when present, is a D-amino acid.

15. The peptide of any one of Claims 1-14 wherein the terminal - C(O)OH of the peptide is replaced with -C(O)R₂, wherein:
R₂ is―̅NH₂, -NHR₁, -N(R₁)₂, -OR₁, or R₁; and
R₁, is an alkyl, aryl, or heteroaryl.

16. A pharmaceutical composition comprising a pharmaceutically-acceptable carrier and a peptide or physiologically-acceptable salt thereof according to any one of Claims 1-15.

17. A kit comprising a container holding a peptide or physiologically-acceptable salt thereof according to any one of claims 1-15.

18. A peptide or a physiologically-acceptable salt thereof according to any one of Claims 1-15 for use in reducing the concentration of a metal ion in an animal in need thereof.

19. A peptide or a physiologically-acceptable salt thereof according to any one of Claims 1-15 for use in reducing the damage done by reactive oxygen species in an animal in need thereof.

20. A peptide or a physiologically-acceptable salt thereof according to any one of Claims 1-15 for use in reducing the damage done by reactive oxygen species in an organ or tissue that has been removed from an animal.

21. A peptide or a physiologically-acceptable salt thereof according to any one of Claims 1-15 for use in treating a disease or condition selected from the group consisting of adult respiratory distress syndrome, aging, AIDS, artherosclerosis, arthritis, asthma, autoimmune diseases, cancer, carcinogenesis, cellular damage caused by ionizing radiation, chronic granulomatous disease, cirrhosis, Crohn's disease, diabetes, eye diseases, emphysema, inflammation, ischemia, neoplastic diseases, neurological trauma, neurodegenerative diseases, pancreatitis, peripheral vascular disease, pulmonary embolism, renal disease, reperfusion, shock, tissue damage occurring upon administration of chemotherapeutics, tissue damage after surgery, toxic reactions, traumatic crush injury and Wilson's disease.


SEQUENCE LISTING

<110> Bar-Or, David
      Curtis, C. G.
      Lau, Edward
      Rao, Nagarajo K.R.
      Winkler, James V.

<120> METAL BINDING COMPOUNDS AND USES THEREFOR

<130> 4172-3-PCT

<140> PCT/US00/26952
<141> 2000-09-29

<150> 60/157,404
<151> 1999-10-01

<150> 60/211,078
<151> 2000-06-13

<160> 6

<170> PatentIn Ver. 2.1

<210> 1
<211> 4
<212> PRT
<213> Homo sapiens

<400> 1
Asp Ala His Lys
  1


<210> 2
<211> 8
<212> PRT
<213> Artificial Sequence

<220>
<223> Description of Artificial Sequence:metal

<220>
<221> METAL
<222> (1)..(4)
<223> copper, nickel and other transition metals

<220>
<221> METAL
<222> (5)..(8)
<223> copper, nickel and other transition metals

<220>
<221> VARIANT
<222> (8)
<223> Xaa = Orn

<400> 2
Asp Ala His Gly Gly His Ala Xaa
  1               5

<210> 3
<211> 12
<212> PRT
<213> Homo sapiens

<400> 3
Asp Ala His Lys Ser Glu Val Ala His Arg Phe Lys
 1               5                   10


<210> 4
<211> 11
<212> PRT
<213> Homo sapiens

<400> 4
Ala His Lys Ser Glu Val Ala His Arg Phe Lys
 1               5                   10


<210> 5
<211> 10
<212> PRT
<213> Homo sapiens

<400> 5
His Lys Ser Glu Val Ala His Arg Phe Lys
 1               5                   10


<210> 6
<211> 12
<212> PRT
<213> Artificial Sequence

<220>
<221> MOD_RES
<222> (1)
<223> ACETYLATION

<220>
<223> Description of Artificial Sequence:variant

<400> 6
Asp Ala His Lys Ser Glu Val Ala His Arg Phe Lys
 1               5                   10